# EUROPEAN PATENT APPLICATION

(11) **EP 1 201 250 A1**
(43) Date of publication of application: **02.05.2002**
(21) Application number: 00203724.0
(22) Date of filing: 25.10.2000
(51) Int. Cl.: A61K 39/39, A61K 39/015

(54) **Immunogenic compositions comprising liver stage malarial antigens**

(71) Applicant: SMITHKLINE BEECHAM BIOLOGICALS S.A., 1330 Rixensart (BE); INSTITUT PASTEUR, 75724 Paris Cédex 15 (FR)
(72) Inventor: Cohen, Joe, Smithkline Beecham Biologicals s.a., 1330 Rixensart (BE); Druilhe, Pierre, 75724 - Paris Cedex 15 (FR)
(74) Representative: Tyrrell, Arthur William Russell, Dr.

(57) **Abstract**

A vaccine composition comprising a Th1-inducing adjuvant in combination with a protecting Liver Stage Antigen or immunological fragment thereof of a human malaria parasite, especially Plasmodium falciparum, with the proviso that when the immunological fragment is an immunological fragment of LSA-3 the Th1-inducing adjuvant is not Montanide. In a preferred aspect the Th1-inducing adjuvant comprises QS21, De-O-acylated monophosphoryl lipid A (3D-MPL) and an oil in water emulsion wherein the oil in water emulsion has the following composition: a metabolisible oil, such a squalene, alpha tocopherol and tween 80. In a further preferred aspect the protecting Liver Stage Antigen is Liver Stage Antigen 3 (LSA-3) or an immunological fragment thereof. A multivalent vaccine composition is also provided comprising the vaccine composition of the invention and in addition at least one other protecting antigen or an immunological fragment thereof, of a malaria parasite.

## Description

The present invention relates to novel vaccine formulations, to methods of their production and to their use in medicine. In particular, the present invention relates to a malaria antigen known as Liver Stage Antigen 3 in association with an oil in water emulsion. Such emulsions comprise tocopherol, squalene, Tween 80, Span 85 and Lecithin and have useful adjuvant properties. Vaccines containing QS21, an Hplc purified non-toxic fraction derived from the bark of Quillaja Saponaria Molina, and/or 3 De-O-acylated monophosphoryl lipid A (3 D-MPL), together with such oil in water emulsions also form part of the invention. Other aspects of the invention are described hereinbelow.

It has long been known that enterobacterial lipopolysaccharide (LPS) is a potent stimulator of the immune system, although its use in adjuvants has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419).

A further detoxified version of MPL results from the removal of the acyl chain from the 3-position of the disaccharide backbone, and is called 3-O-Deacylated monophosphoryl lipid A (3D-MPL). 3 De-O-acylated monophosphoryl lipid A is known from GB2 220 211 (Ribi). Chemically it is a mixture of 3 De-O-acylated monophosphoryl lipid A with 4, 5 or 6 acylated chains and is manufactured by Ribi Immunochem Montana. GB 2122204B also discloses the preparation of diphosphoryl lipid A, and 3-O-deacylated variants thereof. Other purified and synthetic lipopolysaccharides have been described (US 6,005,099 and EP 0 729 473 B1; Hilgers *et al*., 1986, *Int.Arch.Allergy.Immunol*., 79(4):392-6; Hilgers *et al*., 1987, Immunology, 60(1):141-6; and EP 0 549 074 B1).

A preferred form of 3 De-O-acylated monophosphoryl lipid A (3D-MPL) is in the form of an emulsion having a small particle size less than 0.2µm in diameter, disclosed in International Patent Application No. WO 92/116556 (SmithKline Beecham Biologicals s.a.). See also WO 94/21292.

Aqueous formulations comprising monophosphoryl lipid A and a surfactant have been described in WO98/43670A2.

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., *Crit Rev Ther Drug Carrier Syst,* 1996, 12 (1-2):1-55; and EP 0 362 279 B1. Particulate structures, termed Immune Stimulating Complexes (ISCOMS), comprising fractions of Quil A are haemolytic and have been used in the manufacture of vaccines (Morein, B., EP 0 109 942 B1; WO 96/11711; WO 96/33739). The haemolytic saponins QS21 and QS17 (HPLC purified fractions of Quil A) have been described as potent systemic adjuvants, and the method of their production is disclosed in US Patent No.5,057,540 and EP 0 362 279 B1. Other saponins which have been used in systemic vaccination studies include those derived from other plant species such as Gypsophila and Saponaria (Bomford *et al*., Vaccine, 10(9):572-577, 1992).

QS21 is a Hplc purified non toxic fraction of a saponin from the bark of the South American tree Quillaja Saponaria Molina and its method of its production is disclosed (as QA21) in US patent No. 5,057,540.

Oil emulsion adjuvants have been known for many years, including work on Freund's complete and incomplete mineral oil emulsion adjuvants. Since that time much work has been performed to design stable and well tolerated alternatives to these potent, but reactogenic, adjuvant formulations.

Many single or multiphase emulsion systems have been described. Oil in water emulsion adjuvants *per se* have been suggested to be useful as adjuvant compositions (EP O 399 843B), also combinations of oil in water emulsions and other active agents have been described as adjuvants for vaccines (WO 95/17210). Other oil emulsion adjuvants have been described, such as water in oil emulsions (US 5,422,109; EP 0 480 982 B2) and water in oil in water emulsions (US 5,424,067; EP 0 480 981 B).

In order for any oil in water composition to be suitable for human administration, the oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts (such as peanut oil), seeds, and grains are common sources of vegetable oils. Synthetic oils are also part of this invention and can include commercially available oils such as NEOBEE® and others. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil virtue of the fact that it is an intermediate in the biosynthesis of cholesterol (Merck index, 10th Edition, entry no.8619).

The oil in water emulsions which form part of the present invention when formulated with 3 D-MPL and QS21 are preferential stimulators of IgG2a production and TH1 cell response. This is advantageous, because of the known implication of TH₁ response in cell mediated response. Indeed in mice induction of IgG2a is correlated with such an immune response.

The observation that it is possible to induce strong cytolytic T lymphocyte responses is significant as these responses, in certain animal models have been shown to induce protection against disease.

The present inventors have shown that the combination of the adjuvants QS21 and 3D-MPL together with an oil in water emulsion with an antigen results in a powerful induction of CS protein specific CTL in the spleen. QS21 also enhances induction of CTL on its own, while 3D-MPL does not.

Induction of CTL is easily seen when the target antigen is synthesised intracellularly (e.g. in infections by viruses, intracellular bacteria, or in tumours), because peptides generated by proteolytic breakdown of the antigen can enter the appropriate processing pathway, leading to presentation in association with class I molecules on the cell membrane. However, in general, pre-formed soluble antigen does not reach this processing and presentation pathway, and does not elicit class I restricted CTL. Therefore conventional non-living vaccines, while eliciting antibody and T helper responses, do not generally induce CTL mediated Immunity. The combination of the two adjuvants QS21 and 3D-MPL together with an oil in water emulsion can overcome this serious limitation of vaccines based or recombinant proteins, and induce a wider spectrum of immune responses.

CTL specific for CS protein have been shown to protect from malaria in mouse model systems (Romero et al. Nature 341:323 (1989)). In human trials where volunteers were immunised using irradiated sporozoites of P. falciparum, and shown to be protected against subsequent malaria challenge, induction of CTL specific for CS epitopes was demonstrated (Malik et al. Proc. Natl. Acad. Sci. USA 88:3300 (1991)).

The ability to induce CTL specific for an antigen administered as a recombinant molecules is relevant to malaria vaccine development, since the use of irradiated sporozoites would be impractical, on the grounds of production and the nature of the immune response.

In certain systems, the combination of 3D-MPL and QS21 together with an oil in water emulsion have been able to synergistically enhance interferon γ production.

IFN-γ secretion is associated with protective responses against intracellular pathogens, including parasites, bacteria and viruses. Activation of macrophages by IFN-γ enhances intracellular killing of microbes and increases expression of Fc receptors. Direct cytotoxicity may also occur, especially in synergism with lymphotoxin (another product of TH1 cells). IFN-γ is also both an inducer and a product of NK cells, which are major innate effectors of protection. TH1 type responses, either through IFN-γ or other mechanisms, provide preferential help for IgG2a immunoglobulin isotypes.

RTS is a hybrid protein comprising substantially all the C-terminal portion of the circumsporozoite (CS) protein of P.falciparum linked via four amino acids of the preS₂ portion of Hepatitis B surface antigen to the surface (S) antigen of hepatitis B virus (HBV). The structure of RTS and the molecules from which it is derived is disclosed in International Patent Application Publication Number WO 93/10152. When expressed in yeast RTS is produced as a lipoprotein particle, and when it is co-expressed with the S antigen from HBV it produces a mixed particle known as RTS,S.

Liver Stage Antigens are described in Malaria, Parasite Biology, Pathogenesis and Protection (1998 ASM Press, Washington D.C., edited by Irwin W. Sherman), especially Chapter 34 (P. Druilhe et al.).

A 26-amino acid synthetic peptide based on Plasmodium falciparum liver stage antigen 3 (LSA-3) is described in Eur J. Immunol., 1997, 27, 1242-1253 (L. BenMohamed et al).

The immunogenicity of 12 synthetic peptides derived from four new Plasmodium falciparum molecules expressed at pre-erythrocytic stages of the human malaria parasite was reported in Vaccine 18 (2000), pages 2843-2855 (L BenMohamed et al). In these studies the adjuvant Montanide ISA-51 (SEPPIC, Quai D'Orsay, France) was used. There is no report, however, of such peptides being combined with other adjuvants. The present invention is based on the surprising discovery that a Th-1 inducing adjuvant especially an oil in water emulsion which preferably comprises tocopherol, as such or in combination with QS21 and/or 3 D-MPL (or related molecules), enhances immune responses to a defined malaria antigen. Such enhancement available affords better immunological responses than hitherto before.

According to the present invention there is provided a vaccine composition comprising a Th1-inducing adjuvant in combination with a protecting Liver Stage Antigen or immunological fragment thereof of a human malaria parasite with the proviso that when the immunological fragment is an immunological fragment of LSA-3, the Th1-inducing adjuvant is not Montanide.

In a preferred aspect of the invention the Th1-inducing adjuvant comprises QS21, De-O-acylated monophosphoryl lipid A (3D-MPL) and an oil in water emulsion wherein the oil in water emulsion has the following composition: a metabolisible oil, such a squalene, alpha tocopherol and tween 80.

It will be appreciated that variants or derivatives of QS21 and 3-DMPL as described above may also be used without departing from the spirit of the invention.

The bacterial lipopolysaccharide derived adjuvants to be formulated in the adjuvant combinations of the present invention may be purified and processed from bacterial sources, or alternatively they may be synthetic. Accordingly, the LPS derivatives that may be used in the present invention are those immunostimulants that are similar in structure to that of LPS or MPL or 3D-MPL. In another aspect of the present invention the LPS derivatives may be an acylated monosaccharide, which is a sub-portion of MPL. In a preferred aspect the 3-DMPL is small particle 3-DMPL as described in WO 92/116556.

The oil emulsion adjuvants for use in the present invention may be natural or synthetic, and may be mineral or organic. Examples of mineral and organic oils will be readily apparent to the man skilled in the art based on the description hereinabove.

Particularly preferred oil emulsions are oil in water emulsions, and in particular squalene in water emulsions.

In addition, the most preferred oil emulsion adjuvants of the present invention comprise an antioxidant, which is preferably the oil α-tocopherol (vitamin E, EP 0 382 271 B1).

WO 95/17210 discloses emulsion adjuvants based on squalene, α-tocopherol, and TWEEN 80, optionally formulated with the immunostimulants QS21 and/or 3D-MPL.

The size of the oil droplets found within the stable oil in water emulsion are preferably less than 1 micron, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions of the present invention are conventionally in the range of from 2 to 10% oil, such as squalene; and when present, from 2 to 10% alpha tocopherol; and from 0.3 to 3% surfactant, such as polyoxyethylene sorbitan monooleate. Preferably the ratio of oil: alpha tocopherol is equal or less than 1 as this provides a more stable emulsion. Span 85 may also be present at a level of about 1%. In some cases it may be advantageous that the vaccines of the present invention will further contain a stabiliser. Preferably the oil emulsion contains a surfactant such as polyoxyethylene sorbitan monooleate (TWEEN80™), but it will be clear to the man skilled in the art that other surfactants may be used, preferred examples of which are the SPAN series (especially SPAN85) and or lecithin.

The method of producing oil in water emulsions is well known to the man skilled in the art. Commonly, the method comprises the mixing the oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S microfluidics machine, maximum of 50 passes, for a period of 2 minutes at maximum pressure imput of 6 bar (output pressure of about 850 bar)) could be adapted by the man skilled in the art to produce smaller or larger volumes of emulsion. This adaptation could be achieved by routine experimentation comprising the measurement of the resultant emulsion until a preparation was achieved with oil droplets of the required diameter.

In a preferred aspect of the invention the human malaria parasite is Plasmodium falciparum.

In a particular aspect of the invention the said protecting Liver Stage Antigen is the Liver Stage Antigen 3 (LSA-3) or immunological fragment thereof.

However other Liver Stage Antigens may also be used, for example LSA-1 and LSA-2 as described in Malaria, Parasite Biology, Pathogenesis and Protection (1998 ASM Press, Washington D.C., edited by Irwin W. Sherman), especially Chapter 34 (P. Druilhe et al.).

By immunological fragment is meant herein a molecule which has a related or similar sequence to the reference antigen in terms of % homology and which can induce a similar immune response, cellular or humoral, in vivo.

The LSA-3 antigen and polypeptide molecules containing at least 10 consecutive amino acids of the amino acid sequence representing LSA-3 are described in WO 96/41877. LSA-3 for use in the present invention may suitably be prepared as described in the examples section of the present specification. Reference may also be made to C Marchand and P Druilhe, Bulletin of the World Health Organisation, Volume 68 (Suppl.) 158-164 (1990) and US Patent Number 6,100,067.

In a further aspect there is provided a vaccine composition according to the invention comprising in addition at least one other protecting antigen or an immunological fragment thereof, of a malaria parasite, in particular LSA-3.

In particular, the other malaria antigen may be selected from the following group:
a) a hybrid protein comprising substantially all the C-terminal portion of the CS protein, four or more tandem repeats of the immunodominant region, and the surface antigen from hepatitis B virus (HBsAg), in particular RTS,S, or an immunogenic derivative including fragments thereof;
b) the TRAP protein of the T9/96 isolate of Plasmodium falciparum and proteins having at least 80% homology thereto and immunogenic derivatives including fragments thereof (see European Patent Application No 91903249.0);
c) the MSP-1 of Plasmodium falciparum or Plasmodium vivax and proteins having at least 80% homology thereto and immunogenic derivatives including fragments thereof; and
d) the MSP-3 of Plasmodium falciparum or Plasmodium vivax and proteins having at least 70% homology with the C-terminal region thereof, and immunogenic derivatives including fragments thereof.

MSP-1 of P.falciparum or P.vivax is described in US Patent No. 4,837,016. Immunogenic derivatives include fragments thereof such as the C-terminal 42 KDa antigen (p42).

The MSP-3 antigen is described in US Patent Number 6,017,538.

Homology in sequence analysis may be established by the use of Blast 2.0 and Fasta default settings of the algorithms used by these programs. The comparison of LSA-3 sequences in various isolates or stocks can be done using a calculation manual.

By C-terminal region of MSP-3 is meant a 185 amino acid region from positions 193 to 381. It contains a leucine zipper on its extremity (C-terminus part) and is rich in acidic amino acids. The three-dimensional structure is coil-coiled. The clone DG 210 (amino acids 193-257) corresponds to a globular region of high complexity and is followed by the coil-coiled region.

Normally the vaccine composition according to any aspect of the invention invokes a T cell response in a mammal to the antigen or antigenic composition and is preferably capable of stimulating interferon γ production.The oil in water emulsion used in the present invention may be utilised on its own or with other adjuvants or immunostimulants and therefore an important embodiment of the invention is an oil in water formulation comprising squalene or another metabolisable oil, alpha tocopherol, and tween 80. The oil in water emulsion may also contain span 85 and/or Lecithin.

The combination of the two adjuvants QS21 and 3D-MPL together with an oil in water emulsion is particularly preferred. This is known and referred to herein as SBAS2.

The ratio of QS21 : 3D-MPL will typically be in the order of 1 : 10 to 10 : 1; preferably 1 : 5 to 5 : 1 and often substantially 1 : 1. The preferred range for optimal synergy is 2.5:1 to 1:1 3D MPL: QS21. Typically for human administration QS21 and 3D MPL will be present in a vaccine in the range 1 µg - 100 µg, preferably 10 µg - 50 µg per dose. Typically the oil in water will comprise from 2 to 10% squalene, from 2 to 10% alpha tocopherol and from 0.3 to 3% tween 80. Preferably the ratio of squalene: alpha tocopherol is equal or less than 1 as this provides a more stable emulsion. Span 85 may also be present at a level of 1%. In some cases it may be advantageous that the vaccines of the present invention will further contain a stabiliser.

In a further aspect of the present invention there is provided a vaccine as herein described for use in medicine.

In yet a further aspect the invention provides a process for making a vaccine composition according to any aspect of the present invention by mixing the required components using standard techniques. Vaccine preparation is generally described in New Trends and Developments in Vaccines, edited by Voller et al., University Park Press, Baltimore, Maryland, U.S.A. 1978.

Preferably the process comprises admixing QS21, 3D-MPL and the oil in water emulsion with a protecting Liver Stage Antigen of a human malaria parasite as hereinabove defined, optionally with an additional malaria antigen.

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000ug of protein, preferably 2-100 ug, most preferably 4-40 ug. An optimal amount for a particular vaccine can be ascertained by standard studies involving observation of appropriate immune responses in subjects. Following an initial vaccination, subjects may receive one or several booster immunisation adequately spaced.

The formulations of the present invention maybe used for both prophylactic and therapeutic purposes.

Accordingly in one aspect, the invention provides a method of treatment comprising administering an effective amount of a vaccine of the present invention to a patient.

The following examples illustrate the invention.

### Examples

### Example 1

Two adjuvant formulations were made each comprising the following oil in water emulsion component.

SB26: 5% squalene 5% tocopherol 0.4% tween 80; the particle size was 500 nm size SB62: 5% Squalene 5% tocopherol 2.0% tween 80; the particle size was 180 nm

### 1(a) Preparation of emulsion SB62 (2 fold concentrate)

Tween 80 is dissolved in phosphate buffered saline (PBS) to give a 2% solution in the PBS. To provide 100 ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M110S microfluidics machine. The resulting oil droplets have a size of approximately 180 nm.

### 1(b) Preparation of emulsion SB26

This emulsion was prepared in an analogous manner utilising 0.4% tween 80.

To the emulsion of 1 a) or b) an appropriate amount of LSA-3 (for example 2µg to 100µg) may be added and mixed. This may be combined with, for example, 50µg/ml of 3D-MPL and 20µg/ml of QS21 (or related molecules) to give the final formulation.

### Example 2

### Protection against Plasmodium falciparum malaria in chimpanzees by immunisation with a conserved pre-erythrocytic antigen, LSA-3

The basis of the strong immunological protection induced in humans by vaccination with radiation-attenuated pre-erythrocytic malaria parasites is poorly understood. However it is now suspected that the transformation of the irradiated sporozoites into live but developmentally arrested intra-hepatic liver trophozoites is required to induce protection⁹. This occurs at low (15-20 krad) but not at high (23-30 krad) irradiation doses^{9,10}. We reasoned that the differential response of hosts immunised with such irradiated sporozoites could provide a screen for molecules relevant to protection. We proceeded to screen 120 phage lambda clones previously identified as expressing *P*. *falciparum* polypeptides that are expressed during pre-erythrocytic stage parasite development^{6,7} and which derive from ca. 20 distinct genes^{6,7,11,12}. A clone corresponding to each of these putative genes was screened using eight sera from human volunteers (4/6 protected) and from chimpanzees (1/2 protected) immunised with sporozoites irradiated at low or high doses. A single clone (DG729) reacted only with sera from protected humans and chimpanzees. This differential reactivity was further confirmed with a peptide derived from this fragment (Table I). This led us to select this clone for further investigation.

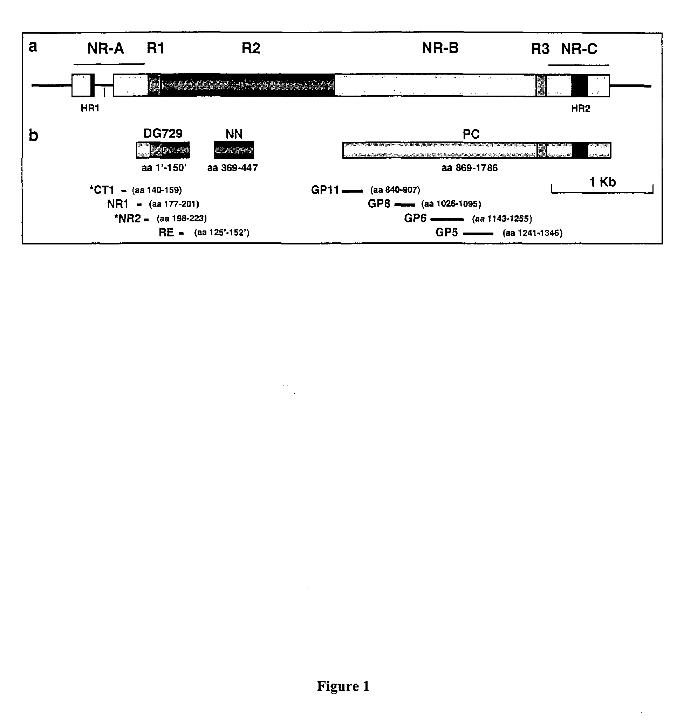

DG729 was used to probe a *P. falciparum* (K1) genomic library. One clone was found to contain the whole gene corresponding to DG729, and which was named Liver Stage Antigen-3 (LSA-3). Full description of the sequence, expression, location and conservation of the *lsa-3* gene is provided in the Supplementary Information (S.I.) and is summarised below and in Figures 1-3. Briefly we identified a single-copy gene which comprises a mini-exon 1, a mini-intron, and a large exon 2 (Fig. 1a), a structure similar to that of other surface antigens of *P. falciparum*¹³. It was recently confirmed that *lsa-3* is located on chromosome 2¹⁴, where the gene was annotated as « RESA-H3 » gene (Acc. Number AE001424). LSA-3, with a predicted molecular weight of 200 kDa (in K1), is made up of large non-repeated sequences flanking three glutamic acid-rich repeated regions, a feature that extends the known *P. falciparum* Glu-rich antigen network¹⁵ to include a pre-erythrocytic component. The location of the original fragment (DG729) and of the peptides corresponding to the repeat region R2 and to the non-repetitive regions NR-A and NR-B are shown in Fig. 1b. Naturally- or artificially- induced antibodies against the non-repeated peptides and the recombinant protein GST-PC were not cross-reactive with the repeated Glu-rich regions, and were used for further studies.

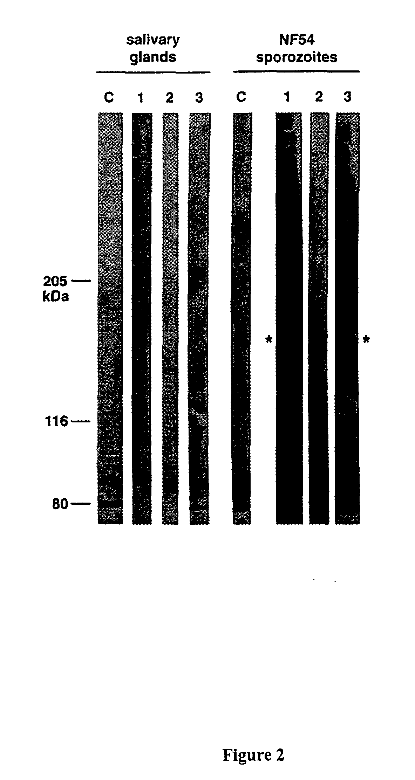

Pre-erythrocytic expression of LSA-3 (see Fig. 2-3 and see S.I.) was confirmed a) by RT-PCR (primers i1 and i2) of total RNA and Western blotting of protein extracts, isolated in both cases from sporozoites, and b) by immunofluorescence antibody test (IFAT) on infected liver sections and dry or wet sporozoite preparations, using antibodies to a non-crossreactive portion of the protein. In the five and six day-old liver schizonts, LSA-3 was located in the parasitophorous vacuole and at the periphery of maturing hepatic merozoites. This location is consistent with the molecular structure of this protein, which contains two hydrophobic regions (Fig. 1a). In our hands, mRNA from *lsa-3* could not be detected in Northern blotted RNA from erythrocytic stages. Western blottings and IFAT of infected red blood cells were also consistently negative with non cross-reactive antibodies. Reactivity was however obtained when antibodies to the Glu-rich repeat region were used. This might explain in part the detection of a putatively homologous antigen (D260) previously described in intra-erythrocytic parasites, and which was identified solely using antibodies which cross-react extensively with Glu-rich epitopes¹⁶.

Polymorphism of many malaria vaccine candidate molecules is of recognised concern, we therefore investigated naturally occurring sequence variation in LSA-3 (see S.I.). The gene was consistently detected by PCR amplification of the NR-A region (primers S1 and S2) in a total of 111 *P. falciparum* isolates, strains or clones of various geographical origin. Using LSA-3 specific antibodies in IFAT assays, the expression of LSA-3 was also detected in liver schizonts of two distinct strains and in all the sporozoites from 30 wild isolates which developed in mosquitoes fed *in vitro* on Thai gametocytes. The repeat regions R1 and R3 are highly conserved, but variation in the number and order of the repeat units of R2 was found to occur amongst different parasite lines. This did not however affect the predicted conserved ?-helical organisation, a secondary structure considered to be important in defining major B-cell epitopes since antibodies which recognise R2 did indeed react positively by IFAT with all the parasites tested. The non-repeated portions of exon 2, where numerous Th and CTL epitopes are found¹⁷⁻¹⁹, displayed a remarkable degree of amino acid (aa) sequence conservation between different parasites (>95.5% homology). The sequence of NR2 peptide was fully conserved amongst K1 and T9/96 parasites, the source of the immunising proteins, the NF54 parasites used for sporozoite challenges, and 27 *P. falciparum* samples of various geographical origin¹⁷. An HLA-B53 restricted epitope identified in the NR-B region of LSA-3 (present in GST-PC recombinant protein) was also found to be free of variation in clone 3D7 and in 18 Gambian isolates¹⁹. This conservation of immunologically important epitopes contrasts with substantial polymorphism in current pre-erythrocytic vaccine candidates.

We selected the chimpanzee to investigate the protective capacity of LSA-3 immunisation for the following reasons. The chimpanzee is the only non-human primate fully susceptible to complete intra-hepatic development of *P. falciparum*, with a comparable rate of sporozoite transformation to liver forms to that seen in humans⁹. The chimpanzee is also the most closely related animal to humans (98.4 % homology at the DNA level ⁸), and one in which detailed investigations of immune responses can be performed and legitimately compared with those of humans ^{17,18}. The fact that parasitological and immunological events can be directly examined in the liver biopsies, a possibility excluded for infected humans, is clearly of considerable significance. A number of preliminary stringent tests were conducted in control animals in order to validate the suitability of this model for vaccine evaluation. Since cost and ethical considerations preclude the use of large number of animals, high reproducibility of the infection in this model system is critical. In a preliminary experiment (Group I, Table II), we confirmed that in the chimpanzee protection by immunisation with irradiated sporozoite is radiation dose-dependent, and we validated the detection of the infected red blood cells as an assay of protection. The results allowed us to define a number of important parameters: a) as in humans, chimpanzees develop a powerful protective response following immunisation with irradiated sporozoite, b) chimpanzees, like humans, remain broadly susceptible to at least five successive challenges, in contrast to lower primates or rodents which become refractory after the first challenge²⁰, and c) as a result of the high dose of inoculated sporozoites detection of erythrocytic parasites corresponded to the first invasion of red cells by merozoites released from intra-hepatocytic schizonts. Positive blood smears were reproducibly obtained in non-protected chimpanzees on days six or seven, In the chimpanzee erythrocytic infections normally remain sub-clinical and self-limiting which was in fact observed despite the high dose challenges. These results have been recently confirmed in two further chimpanzees (Langermans J. *et al*, manuscript in preparation).

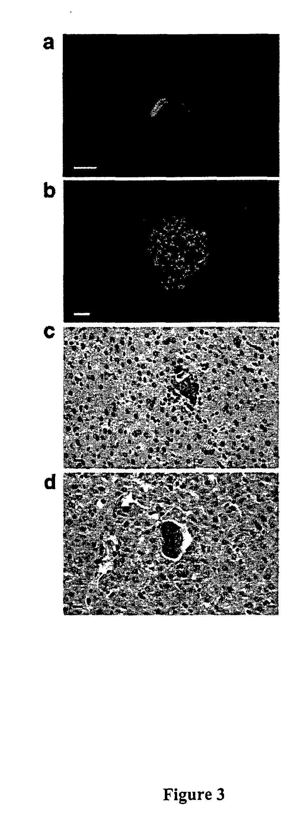

Having established the suitability of the chimpanzee, we proceeded to assay the protective value of LSA-3 immunisation by challenge with viable *P. falciparum* sporozoites. In preliminary experiments, two animals were immunised with a mixture of LSA-3 and LSA-1 recombinant proteins. Full protection against three challenges over several months was only seen in the animal which responded to LSA-3 (both responded to LSA-1). In liver biopsies performed on this animal on day five, only one liver schizont of unhealthy appearance and infiltrated by leukocytes could be detected in the 300 liver sections screened (Dirk, Fig. 3). By contrast 2500 and 750 hepatic schizonts of healthy appearance were observed in the two non-protected controls.

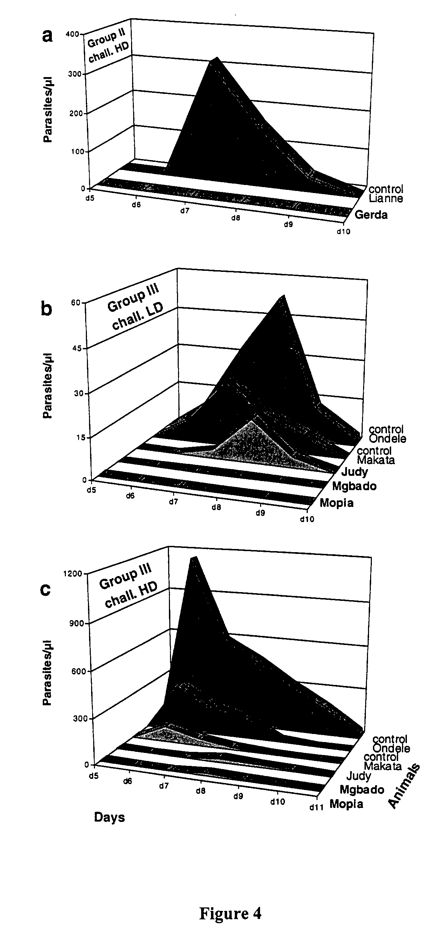

These results led us to focus further immunisation and challenge experiments on LSA-3 alone. Two groups of chimpanzees were used to evaluate lipopeptide and recombinant protein formulations (Table II, Groups II-III). In Group II, one animal (Gerda) was initially immunised solely with the NR2 lipopeptide of LSA-3, and boosted by recombinant LSA-3 molecules in Montanide ISA 51. Gerda was fully protected when challenged with 10⁷ sporozoites, whereas the control receiving Montanide ISA 51 was not (Fig. 4a).

In Gerda boosting with the recombinant LSA-3 formulation was not found to induce any detectable increase in the strong B-cell, T-helper cell and CTL responses already evoked by the initial lipopeptide/peptide injections^{17,18}. We were therefore interested to see whether the simple and well-tolerated peptidic formulation alone could induce protection. Two chimpanzees, Mopia and Mgbado were immunised with LSA-3 lipopeptides/peptides alone (Table II, Group III). Protection against a first challenge with 2 x 10⁴ sporozoites was obtained in both. The same group included an investigation of the effects of microbead presentation of recombinant proteins without adjuvant in one animal (Judy) which resulted in a one-day delay to patency (Fig. 4b). Following a subsequent high dose sporozoite challenge (5 x 10⁶ sporozoites), both Mopia and Mgbado demonstrated a clear two-day delay to patency and a low transient parasitaemia, whilst no protection was found for Judy (Fig. 4c). The delay to patency suggests that the immune responses had caused a reduction exceeding 90% of intra-hepatocytic schizont load²¹ (Fig. 4).

In chimpanzees from groups IV and V, we investigated the efficacy of a less complex lipopeptide mixture alone, or of recombinants adjuvated by SBAS2, a novel adjuvant whose efficacy has been recently established in humans^{4,5}. Since immunogenicity studies^{17,18} and analysis of previous chimpanzee data had indicated that peptide CT1 was poorly immunogenic and thus might not be critical, chimpanzee Patty was immunised by a mix of three instead of four peptides. This animal showed protection upon challenge. Among four animals receiving SBAS2 adjuvated LSA-3 proteins, two showed full, sterile protection against a medium dose challenge. One showed a delay in patency which may be indicative of partial protection, whereas neither the fourth nor the control receiving SBAS2 adjuvant alone were protected. One of the two fully protected chimpanzees was further challenged with a high dose three months later and still showed full protection.

We present here the first description of protective vaccination against human malaria in the chimpanzee. This model provided us with convincing evidence that LSA-3 of *P. falciparum* is a valuable candidate for effective vaccination against pre-erythrocytic stages. A total of nine animals were immunised using lipopeptides in saline or polypeptides in either Montanide or SBAS2 adjuvants. Full sterile protection was induced in six of these nine chimpanzees on first challenge. If the significant delay as compared to controls is taken in consideration, a protective effect induced by LSA-3 was shown in eight of nine animals. Out of the 14 challenges which were performed, complete protection was obtained in seven, and partial protection in an additional four challenges. All seven control animals employed in these studies showed a consistent pattern in the appearance and the course of the blood-stage parasitaemiae following each of the 12 challenges with viable parasites. Demonstration of this reproducibility in controls, in animals immunised by over-irradiated sporozoites, and in an additional 26 challenges performed in other experiments (not shown), is an essential point in the interpretation of our data.

It is encouraging that protection was induced against a heterologous challenge (NF54) in outbred animals immunised with LSA-3 molecules whose sequences were derived from K1 and T9/96 parasites. A variety of immunisation strategies were investigated in the course of this work. The data underpin the value of the SBAS2 adjuvant. The results with Gerda, Mopia, Mgbado and Patty are also particularly encouraging since they are based on simple peptide and lipopeptide formulations which are relatively easy to produce under GMP conditions²². In our animals no local or general reactions was detected following lipopeptide injections, an observation consistent with previous experience with similar formulations derived from SIV in macaques²³ and HbS²⁴ or HIV²² in humans. This bodes well for future clinical trials.

### METHODS

**Selection of clone DG729.** Dot blot analysis of the β-galactosidase-fused recombinant proteins encoded by the pre-erythrocytic clones was performed on nitrocellulose as previously described⁷, using 1/100 diluted human and chimpanzee sera. ELISA was performed in duplicate as previously described²⁵ on 1/100 diluted sera using coating solutions of 0.3, 3 and 10 µg/ml ofNR1, NR2 and RE peptides respectively, in PBS.
**LSA-3 cloning and characterisation.** Detailed description of molecular methods, gene cloning, sequence data, protein characteristics and description of the recombinant proteins and of the peptides are provided in the S.I. The primers used for PCR: S1 (nucl.161-184)/S2 (nucl.454-432) and for RT-PCR: i1 (nucl.695-722)/i2 (nucl.824-799), numbering refers to the *lsa-3* sequence of K1 (Accession Nber AJ007010). All mouse sera used for the Western blot (at dilution 1/100) presented in Fig. 2 were obtained following 3 subcutaneous injections of the immunogen (100 µg) emulsified in SBAS2 adjuvant⁴. Long synthetic peptides GP5, GP6, GP8 and GP11 were synthesised as described in ref. 26 (see Fig. 1 for position).
**Immunogens injected in chimpanzees.** Sequences of the various immunogens evaluated here consisted of clone DG729 and inserts NN and PC, as well as peptides (pep.) NR1, NR2, RE and CT1; their location is shown in Fig. 1 and described in more details in the S.I. Clone DG729, as well as inserts NN and PC were expressed as glutathione-S-transferase-fused recombinants and purified according to manufacturer recommendations (Invitrogen, The Netherlands). Recombinants GST-DG729, -NN and -PC were designed so as to cover 95% of the LSA-3 antigen and were used as a mixture mentioned as LSA-3 GST-rec. Peptides NR1, NR2 and CT1, were also synthesised as palmitoyl-conjugated lipopeptides (lipopep.), as described in ref. 17. Combination of synthetic compounds (mentioned as (lipo)pep.) consisted in a mixture of NR1, NR2 and CT1 lipopeptides and of RE peptide. All peptides and lipopeptides were purified to >90% purity by reversed-phase chromatography, and the impurities consisted essentially of related peptides of shorter sequences¹⁷.
**Chimpanzee immunisations and challenges.** None of the chimpanzees included in this study had previously been exposed to malaria infections or malarial antigens.
Recombinant and synthetic compounds were injected subcutaneously, at a dose of 100 µg for each peptide and/or lipopeptides, and/or 50 µg for each protein. Lipopeptides were always injected in PBS and, except when mentioned, peptides and recombinants were emulsified in Montanide ISA51. Group I animals (Carl and Japie) were immunised by five intra-venous injections of 5 x 10⁶ gamma-irradiated sporozoites at day 0 and weeks 8, 24, 44 and 65, and received three challenges at weeks 71, 97 and 123 (challenge doses are given in Table II). One year after the three challenges reported here, these chimpanzees were re-immunised once, and received one low and one high dose challenges, which revealed the same pattern of protection (not shown, Langermans J. *et al*., manuscript in preparation). In Group II, Gerda received NR2 lipopeptide at day 0 and weeks 3, 13 and 31 as described in ref. 17. She was then boosted with the mixture of LSA-3 GST-rec. at weeks 40, 45, 48 and 50. Control animal Lianne received Montanide ISA51. Challenges were performed at week 60. Group III animals were immunised at day 0 and weeks 3 and 6. Mopia and Mgbado received LSA-3 (lipo)peptides whereas Judy was injected with LSA-3 GST-rec. adsorbed to latex microbeads. Challenges LD and HD were performed at weeks 21 and 29. In Group IV, Patty received LSA-3 (lipo)peptides, but without lipopeptide CT1, whereas Wendy and Willy were injected with LSA-3 GST-rec in SBAS2 adjuvant^{4,5}. Control animal Helen received SBAS2 adjuvant only. All animals were immunized at weeks 0, 4 and 8 and were challenged with 20,000 sporozoites at week 13. In Group V, Cindy and Marty were both immunised at weeks 0, 4, 8 and 26 with LSA-3 GST-rec in SBAS2 adjuvant (as in Group IV) and negative control animal Fauzi received over-irradiated sporozoites similarly to Japie (Group I) at weeks 5, 8, 11 and 26. Challenges LD and HD were performed at weeks 33 and 46 in all three animals.
NF54 sporozoites were obtained from dissected salivary glands of infected *Anopheles gambiae* as previously described²⁷. Sporozoites were pooled, resuspended in PBS and injected intravenously. All animals in each group were challenged with the same pool of sporozoites. For cost reasons, extensive evaluation of the Minimal Infective Dose has not been undertaken, however challenge with 5 x 10³ sporozoites, the lowest dose used to date, has proven infective in four other animals (Thomas, A.W., unpublished data).
**Determination of the protective status.** For Groups I, II, IV and V, animals blood was taken on days five to nine, and evaluated by thick and thin film Giemsa-stained preparations, and confirmed in all cases by *in vitro* culture (not shown), as described in ref. 21. For Group III chimpanzees blood taken every day from day five up to day 18, then every other day up to day 30, was used to prepare thin and thick smears which were Giemsa-stained and examined by two separate microscopists. A chimpanzee was considered a) totally protected when no parasites could be detected in the circulation blood, by direct microscopical observation and by long term culture, or b) partially protected when time to patency was delayed by one or more days as compared to that observed in control animals. In mice, these delays correspond to a protection of 80% (24h) or 96% (48h) against sporozoite challenges. In humans, a 12 hour delay was calculated to correspond to a 92% reduction of liver forms following sporozoite challenges²¹. In a limited number of animals a liver biopsy was performed under anaesthesia by a veterinary doctor on day five following a high dose challenge. Material was fixed and 4 µm sections were made and stained by Giemsa-collophonium28 before complete microscopic enumeration of the liver forms in 300 sections (average area 0.8 cm²). All animals were curatively treated with chloroquine immediately after the period of observation, and irrespective of their protective status.

### References to Example 2

1. Herrington, D., *et al*. Successful immunization of humans with irradiated malaria sporozoites: humoral and cellular responses of the protected vaccinees. *Am. J Trap*. *Med. Hyg*. **45,** 539-547 (1991).
2. Egan, J.E., *et al*. Humoral immune responses in volunteers immunized with irradiated *Plasmodium falciparum* sporozoites. *Am. J. Trop. Med. Hyg.* **49,** 166-73 (1993).
3. Facer, C.A. & M., Tanner. Clinical trials of malaria vaccines: progress and prospects. *Adv. Parasitol.* **39,** 1-68 (1997).
4. Stoute, J.A., *et al*. A preliminary evaluation of a recombinant circumsporozoite protein vaccine against *Plasmodium falciparum* malaria. *New Engl. J*. *Med*. **336,** 86-91 (1997).
5. Stoute, J.A., *et al.* Long-term efficacy and immune responses following immunization with the RTS,S malaria vaccine. *J Infect. Dis.* **178,** 1139-44 (1998).
6. Guérin-Marchand, C., *et al*. A liver stage-specific antigen of *Plasmodium falciparum* characterized by gene cloning. *Nature*. **329,** 164-167 (1987).
7. Marchand, C. & Druilhe, P. How to select *Plasmodium falciparum* pre-erythrocytic antigens in an expression library without defined probe. *Bull. WHO.* **68 (suppl.),** 158-164 (1990).
8. Miyamoto, M.M., Koop, B. F., Slightom, J. L., Goodman, M. and M.R., Tennant. Molecular systematics of higher primates: genealogical relations and classification. *Proc. Nat. Acad. Sci. U.S.A*. **85,** 7627-31 (1988).
9. Druilhe, P., *et al*. in "Malaria. Parasite Biology, Pathogenesis and Protection" (eds. Irwin W. Sherman), p.513-543 (American Society for Microbiology, Washington D.C., 1998).
10. Mellouk, S., Lunel, F., Sedegah, M., Beaudoin, R.L. and P., Druilhe. Protection against malaria induced by irradiated sporozoites. *Lancet.* 335, **721** (1990).
11. Fidock, D.A., *et al.* Cloning and characterization of a *Plasmodium falciparum* sporozoite surface antigen - STARP. *Mol. Biochem. Parasitol.* **64,** 219-232 (1994).
12. Bottius, E., *et al.* A novel *Plasmodium falciparum* sporozoite and liver stage antigen (SALSA) defines major B, T helper, and CTL epitopes. *J. Immunol.* **156,** 2874-2884 (1996).
13. Kemp, D.J., Cowman, A.F. and D., Walliker. Genetic diversity in *Plasmodium falciparum. Adv. Parasitol*. **29,** 75-149 (1990).
14. Gardner, M.J., *et al*. Chromosome 2 sequence of the human malaria parasite *Plasmodium falciparum*. Science, **282,** 1126-1132 (1998).
15 Moelans, I.I.M.D. & J.G.G., Schoenmakers. Crossreactive antigens between life cycle stages of *Plasmodium falciparum. Parasitol. Today.* **8,** 118-123 (1992).
16. Barnes, D. A., Wollish, W., Nelson, R.G., Leech, J.H. and C., Petersen. *Plasmodium falciparum*: D260, an intraerythrocytic parasite protein, is a member of the glutamic acid dipeptide-repeat family of proteins. *Exp. Parasitol*., **81,** 79-89 (1995).
17. Ben Mohamed, L., *et al*. Lipopeptide immunization without adjuvant induces potent and long-lasting B, T helper, and cytotoxic T lymphocyte responses against a malaria liver stage antigen in mice and chimpanzees. *Eur. J. Immunol*. **27,** 1242-1253 (1997).
18. Ben Mohamed, L. *et al.* High immunogenicity in chimpanzees of peptides and lipopeptides derived from four new *Plasmodium falciparum* pre-erythrocytic molecules. *Vaccine*, **18,** 2843-2855 (2000).
19 Aidoo, M., *et al.* CTL epitopes for HLA-B53 and other HLA types in the malaria vaccine candidate Liver Stage Antigen-3. *Infect. Immun.* 68, 227-232 (2000).
20. Nüssler, A.K., *et al. In vivo* induction of the nitric oxide pathway in hepatocytes after injection with irradiated malaria sporozoites, malaria blood parasites or adjuvants. *Eur. J*. *Immunol*. **23,** 882-887 (1993).
21. Murphy, J.R., Baqar, S., Davis, J.R., Herrington, D.A. and D.F., Clyde. Evidence for a 6.5-day minimum exoerythrocytic cycle for *Plasmodium falciparum* in humans and confirmation that immunization with a synthetic peptide representative of a region of the circumsporozoite protein retards infection. *J*. *Clin. Microbiol*. **27,** 1434-1437 (1989).
22. Gahery-Segard, H., *et al*. Multiepitopic B- and T-cell responses induced in humans by a Human Immunodeficiency Virus type 1 lipopeptide vaccine. *J. Virol*. **4,** 1694-703 (2000).
23. Bourgault, I., *et al.* Simian immunodeficiency virus as a model for vaccination against HIV: induction in rhesus macaques of GAG or NEF specific cytotoxic T lymphocytes by lipopeptides. *J Immunol*. **152,** 2530-2537 (1994).
24. Vitiello, A., *et al*. Development of a lipopeptide-based therapeutic vaccine to treat chronic HBV infection. Induction of a primary cytotoxic T lymphocyte response in humans. *J. Clin. Invest.* **95,** 341-349 (1995).
25. Londoño, J.A., Gras-Masse, H., Dubeaux, C., Tartar, A. and P., Druilhe. Secondary structure and immunogenicity of hybrid synthetic peptides derived from two *Plasmodium falciparum* pre-erythrocytic antigens. *J. Immunol.* **145,** 1557-1563 (1990).
26. Roggero, M.A., *et al.* Synthesis and immunological characterization of 104-mer and 102-mer peptides corresponding to the N- and C-terminal regions of the *Plasmodium falciparum* CS Protein. *Mol. Immunol*. **32,** 1301-1309 (1995).
27. Ponnudurai, T., *et al.* Sporozoite load of mosquitoes infected with *Plasmodium falciparum. Trans. Roy Soc. Trop. Med. Hyg*. **83,** 67-70 (1989).
28. Druilhe, P., Puebla, R.M., Miltgen, F., Perrin, L. and M., Gentilini. Species- and stage-specific antigens in exoerythrocytic stages of *Plasmodium falciparum. Am. J Trop. Med. Hyg.* **33,** 336-341 (1984).
29. Meis, J.F.G.M., *et al. Plasmodium falciparum*: studies on mature exoerythrocytic forms in the liver of the chimpanzee, *Pan troglodytes. Exp. Parasitol.* **70,** 1-11 (1990).

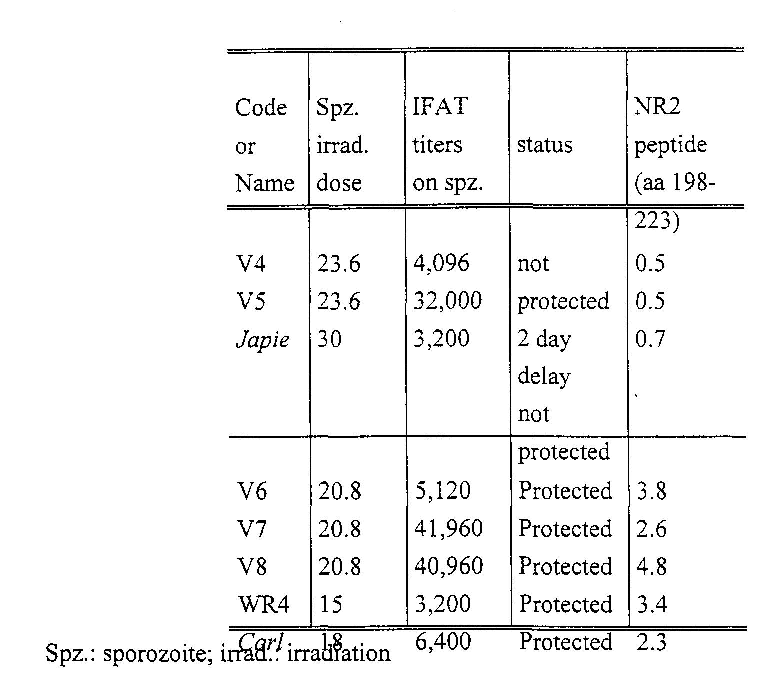

**Table I. Differential reactivity of sera from protected or non-protected humans or chimpanzees with peptide NR2.** IgG-specific antibodies against peptide NR2 were measured by ELISA in sera from human volunteers (codes) and chimpanzees (names in italic) immunised with sporozoites irradiated at low or high dose (in krad). Codes, immunisation schemes, sporozoite IFAT titres and protective status determination for human volunteers V4-V8 and WR4 are detailed in ref. 1 and 2, respectively. Chimpanzees Carl and Japie were immunised and challenged as described in the text and the Methods (Group I). ELISA titres are expressed in arbitrary units representing the ratio of the mean ODs from test sera to the mean OD plus three standard deviations from 10 controls studied in parallel in the same plate. Results are taken as positive for ratios above one (in bold). Similar experiments performed with peptides NR1 and RE (see Fig. 1) yielded negative results with these sera (not shown).

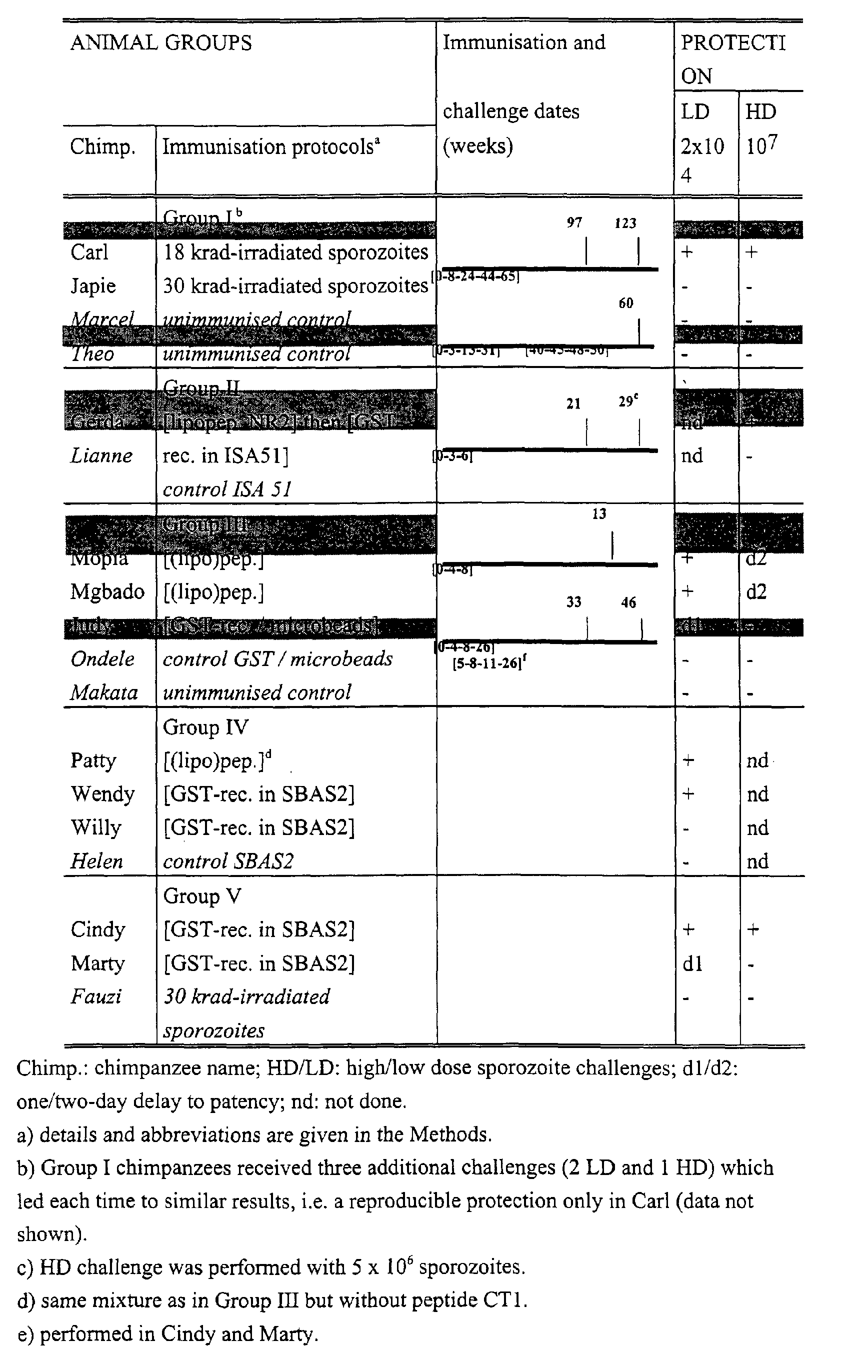

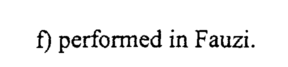

**Table II. Immunisation and challenge experiments in the chimpanzees.** Challenges were performed with either 2x10⁴ (low dose) or 10⁷ (high dose) NF54 *P. falciparum* sporozoites ("Protection" column). Immunisation schedules (in brackets under the bar) and of challenges (indicated by arrows above the bar) are expressed in weeks from first immunisation. Shading highlights protected animals. Complete protection is indicated with (+); a delay to patency (in days) as compared to controls and non-protected animals is indicated by d1 or d2 (determination of the protective status is detailed in the Methods).

### LEGENDS FOR FIGURES

**Figure 1: Schematic representation of the LSA-3 gene, recombinant proteins and peptides. a)** 6.2 Kb *Eco* RI-insert isolated from K1 parasite genomic DNA library that hybridised with DG729. The 5.53 Kb gene comprises a 198 bp exon 1, a 168 bp intron (i) and a 5.16 Kb exon 2. Regions NR-A, -B and -C correspond to non-repeated sequences whereas R1 to R3 designate the three repeat blocks. The two hydrophobic regions potentially corresponding to the NH₂-terminal signal peptide and the anchor region are indicated by HR1 and HR2 respectively. b) Location of the sequences encoding for LSA-3 in the recombinant fusion proteins (first line) and the synthetic peptides (strokes) used in this study (see Supplementary Information for aa numbering). For the immunisations, CT1 and NR2 were also used as palmitoyl-conjugated lipopeptides¹⁷ (indicated by *).

**Figure 2: LSA-3 expression in *P. falciparum* sporozoites.** Western blot analysis was performed on protein extracts from NF54 sporozoites and control uninfected mosquito salivary glands using mouse antisera directed against: C) control GST, 1) GST-PC, 2) peptides GP5, GP6, GP8 or GP11, 3) GST-729 (see Fig. 1, Methods and S.I.). LSA-3 is visualised as a 175 kDa protein (*), in agreement with the theoretical molecular weight of LSA-3 in this parasite strain.

**Figure 3: Immunostaining of *P. falciparum* pre-erythrocytic stages with anti-LSA-3 antibodies.** a) sporozoites stained by IFAT with human antibodies affinity purified on recombinant ßga1-DG729. b) staining by IFAT of day six post-challenge liver stages²⁹ from a chimpanzee, using the antibodies induced by lipopeptide NR2 injection¹⁷ in chimpanzee Gerda (see S.I. for additional pictures). c) The single residual liver schizont detected in a chimpanzee Dirk (day five post-challenge) appeared infiltrated by lymphomononuclear cells, as compared in d) to one of the numerous healthy schizonts observed in the control chimpanzee Peer (total of ca 2500 schizonts/300 liver sections, Giemsa-collophonium staining²⁸) (see text). Bars correspond to 5 µm in panel a) and 20 µm in panels b) to d).

**Figure 4: Blood parasitaemia courses in Groups II and III.** a) chimpanzees from Group II and b-c) animals in Group III, following high dose (HD) or low dose (LD) challenges with NF54 sporozoites. Names of totally or partially protected animals are in bold. Hatched patterns correspond to control chimpanzees. Parasitaemia scales are different for each challenge, as expected from challenges with different numbers of sporozoites. Note that the day of patency in control and non-protected animals was the same for a given challenge inoculum within each group (in the above and in other groups not shown here).

### Example 3

### Sequence data and supplementary information

The following further information exemplifying the invention is supplied:
**Sequence Data -** Gene: full Sequence (K1 parasite)
   - Protein: full Sequence (K1 parasite)
   - Clones DG729 / DG679 (T9/96 parasite)
   - Note on LSA-3 sequence in parasite 3D7
**Gene & Protein -** Structure . Restriction map . Hydrophobicity
   - Oligonucleotides employed
   - Organisation
**Regions & Comments -** NR-A . R1 . R2 . NR-B . R3 . NR-C
**Conservation -** of the gene
   - of the sequence
   - of repeat region R2
   - comparaison of immunising and challenging sequences

### Stage Specificity & Subcellular Location

### Homologies - Intraspecies

- Interspecies

### Synthetic Peptides & Recombinant Proteins used for Chimpanzee Immunisations

- Peptides CT1 . NR1 . NR2 . RE
- Recombinant proteins β-729. GST-729. GST-NN. GST-PC

### Methods

### References to Example 3

SEQUENCE DATA

K1 PARASITE STRAIN- clone k1.2 Accession Nber AJ007010

GENE: full sequence

| 10 | 20 | 30 | 40 | 50 | 60 | 70 | 80 | 90 | 100
1 atgacaaata gtaattacaa atcaaataat aaaacatata atgaaaataa taatgaacaa ataactacca tatttaatag aacaaatatg aatccgataa
101 aaaaatgtca tatgagagaa aaaataaata agtacttttt tttgatcaaa attttgacat gcaccatttt aatatgggct gtacaatatg ataataacgt
201 aagataaaaa actaaataat aaatataaat aaaaaaaaaa aaaaaaaaaa aaaaatcaac tatatagtat gtataatata tatatatata tatatatata
301 tatatatata tatatattta tttttattta tttattaatt tttttttttt tatattatct ttttagtctg atataaacaa gagttggaaa aaaaatacgt
401 atgtagataa gaaattgaat aaactattta acagaagttt aggagaatct caagtaaatg gtgaattagc tagtgaagaa gtaaaggaaa aaattcttga
501 cttattagaa gaaggaaata cattaactga aagtgtagat gataataaaa atttagaaga agccgaagat ataaaggaaa atatcttatt aagtaatata
601 gaagaaccaa aagaaaatat tattgacaat ttattaaata atattggaca aaattcagaa aaacaagaaa gtgtatcaga aaatgtacaa gtcagtgatg
701 aactttttaa tgaattatta aatagtgtag atgttaatgg agaagtaaaa gaaaatattt tggaggaaag tcaagttaat gacgatattt ttaatagttt
801 agtaaaaagt gttcaacaag aacaacaaca caatgttgaa gaaaaagttg aagaaagtgt agaagaaaat gacgaagaaa gtgtagaaga aaatgtagaa
901 gaaaatgtag aagaaaatga cgacggaagt gtagcctcaa gtgttgaaga aagtatagct tcaagtgttg atgaaagtat agattcaagt attgaagaaa
1001 atgtagctcc aactgttgaa gaaatcgtag ctccaagtgt tgtagaaagt gtggctccaa gtgttgaaga aagtgtagaa gaaaatgttg aagaaagtgt
1101 agctgaaaat gttgaagaaa gtgtagctga aaatgttgaa gaaagtgtag ctgaaaatgt tgaagaaagt gtagctgaaa atgttgaaga aatcgtagct
1201 ccaactgttg aagaaatcgt agctccaact gttgaagaaa ttgtagctcc aagtgttgta gaaagtgtgg ctccaagtgt tgaagaaagt gtagaagaaa
1301 atgttgaaga aagtgtagct gaaaatgttg aagaaagtgt agctgaaaat gttgaagaaa gtgtagctga aaatgttgaa gaaagtgtag ctgaaaatgt
1401 tgaagaaagt gtagctgaaa atgttgaaga aatcgtagct ccaactgttg aagaaatcgt agctccaact gttgaagaaa ttgtagctcc aagtgttgta
1501 gaaagtgtgg ctccaagtgt tgaagaaagt gtagaagaaa atgttgaaga aagtgtagct gaaaatgttg aagaaagtgt agctgaaaat gttgaagaaa
1601 gtgtagctga aaatgttgaa gaaagtgtag ctgaaaatgt tgaagaaagt gtagctgaaa atgttgaaga aagtgtagct gaaaatgttg aagaaagtgt
1701 agctgaaaat gttgaagaaa tcgtagctcc aactgttgaa gaaatcgtag ctccaactgt tgaagaaatt gtagctccaa gtgttgtaga aagtgtggct
1801 ccaagtgttg aagaaagtgt agaagaaaat gttgaagaaa gtgtagctga aaatgttgaa gaaagtgtag ctgaaaatgt tgaagaaagt gtagctgaaa
1901 atgttgaaga aagtgtagct gaaaatgttg aagaaatcgt agctccaact gttgaagaaa tcgtagctcc aactgttgaa gaaattgtag ctccaagtgt
2001 tgtagaaagt gtggctccaa gtgttgaaga aagtgtagaa gaaaatgttg aagaaagtgt agctgaaaat gttgaagaaa gtgtagctga aaatgttgaa
2101 gaaagtgtag ctgaaaatgt tgaagaaatc gtagctccaa ctgttgaaga aatcgtagct ccaactgttg aagaaattgt agctccaagt gttgtagaaa
2201 gtgtggctcc aagtgttgaa gaaagtgtag aagaaaatgt tgaagaaagt gtagctgaaa atgttgaaga aagtgtagct gaaaatgttg aagaaagtgt
2301 agctgaaaat gttgaagaaa gtgtagctga aaatgttgaa gaaatcgtag ctccaactgt tgaagaaatc gtagctccaa ctgttgaaga aattgtagct
2401 ccaagtgttg tagaaagtgt ggctccaagt gttgaagaaa gtgtagaaga aaatgttgaa gaaagtgtag ctgaaaatgt tgaagaaagt gtagctgaaa
2501 atgttgaaga aagtgtagct gaaaatgttg aagaaagtgt agctccaact gttgaagaaa ttgtagctcc aagtgttgaa gaaagtgtag ctccaagtgt
2601 tgaagaaagt gttgctgaaa acgttgcaac aaatttatca gacaatcttt taagtaattt attaggtggt atcgaaactg aggaaataaa ggacagtata
2701 ttaaatgaga tagaagaagt aaaagaaaat gtagtcacca caatactaga aaacgtagaa gaaactacag ctgaaagtgt aactactttt agtaacatat
2801 tagaggagat acaagaaaat actattacta atgatactat agaggaaaaa ttagaagaac tccacgaaaa tgtattaagt gccgctttag aaaataccca
2901 aagtgaagag gaaaagaaag aagtaataga tgtaattgaa gaagtaaaag aagaggtcgc taccacttta atagaaactg tggaacaggc agaagaaaag
3001 agcgcaaata caattacgga aatatttgaa aatttagaag aaaatgcagt agaaagtaat gaaaatgttg cagagaattt agagaaatta aacgaaactg
3101 tatttaatac tgtattagat aaagtagagg aaacagtaga aattagcgga gaaagtttag aaaacaatga aatggataaa gcatttttta gtgaaatatt
3201 tgataatgta aaaggaatac aagaaaattt attaacaggt atgtttcgaa gtatagaaac cagtatagta atccaatcag aagaaaaggt tgatttgaat
3301 gaaaatgtgg ttagttcgat tttagataat atagaaaata tgaaagaagg tttattaaat aaattagaaa atatttcaag tactgaaggt gttcaagaaa
3401 ctgtaactga acatgtagaa caaaatgtat atgtggatgt tgatgttcct gctatgaaag atcaattttt aggaatatta aatgaggcag gagggttgaa
3501 agaaatgttt tttaatttgg aagatgtatt taaaagtgaa agtgatgtaa ttactgtaga agaaattaag gatgaaccgg ttcaaaaaga ggtagaaaaa
3601 gaaactgtta gtattattga agaaatggaa gaaaatattg tagatgtatt agaggaagaa aaagaagatt taacagacaa gatgatagat gcagtagaag
3701 aatccataga aatatcttca gattctaaag aagaaactga atctattaaa gataaagaaa aagatgtttc actagttgtt gaagaagttc aagacaatga
3801 tatggatgaa agtgttgaga aagttttaga attgaaaaat atggaagagg agttaatgaa ggatgctgtt gaaataaatg acattactag caaacttatt
3901 gaagaaactc aagagttaaa tgaagtagaa gcagatttaa taaaagatat ggaaaaatta aaagaattag aaaaagcatt atcagaagat tctaaagaaa
4001 taatagatgc aaaagatgat acattagaaa aagttattga agaggaacat gatataacga cgacgttgga tgaagttgta gaattaaaag atgtcgaaga
4101 agacaagatc gaaaaagtat ctgatttaaa agatcttgaa gaagatatat taaaagaagt aaaagaaatc aaagaacttg aaagtgaaat tttagaagat
4201 tataaagaat taaaaactat tgaaacagat atttagaag agaaaaaaga aatagaaaaa gatcattttg aaaaattcga agaagaagct gaagaaataa
4301 aagatcttga agcagatata ttaaaagaag tatcttcatt agaagttgaa gaagaaaaaa aattagaaga agtacacgaa ttaaaagaag aggtagaaca
4401 tataataagt ggtgatgcgc atataaaagg tttggaagaa gatgatttag aagaagtaga tgatttaaaa ggaagtatat tagacatgtt aaagggagat
4501 atggaattag gggatatgga taaggaaagt ttagaagatg taacaacaaa acttggagaa agagttgaat ccttaaaaga tgttttatct agtgcattag
4601 gcatggatga agaacaaatg aaaacaagaa aaaaagctca aagacctaag ttggaagaag tattattaaa agaagaggtt aaagaagaac caaagaaaaa
4701 aataacaaaa aagaaagtaa ggtttgatat taaggataag gaaccaaaag atgaaatagt agaagttgaa atgaaagatg aagatataga agaagatgta
4801 gaagaagata tagaagaaga tatagaagaa gataaagttg aagatataga tgaagatata gatgaagata taggtgaaga caaagatgaa gttatagatt
4901 taatagtcca aaaagagaaa cgcattgaaa aggttaaagc gaaaaagaaa aaattagaaa aaaaagttga agaaggtgtt agtggtctta aaaaacacgt
5001 agacgaagta atgaaatatg ttcaaaaaat tgataaagaa gttgataaag aagtatctaa agctttagaa tcaaaaaatg atgttactaa tgttttaaaa
5101 caaaatcaag atttttttag taaagttaaa aacttcgtaa aaaaatataa agtatttgct gcaccattca tatctgccgt tgcagcattt gcatcatatg
5201 tagttgggtt ctttacattt tctttatttt catcatgtgt aacaatagct tcttcaactt acttattatc aaaagttgac aaaactataa ataaaaataa
5301 ggagagaccg ttttattcat ttgtatttga tatctttaag aatttaaaac attatttaca acaaatgaaa gaaaaattta gtaaagaaaa aaataataat
5401 gtaatagaag taacaaacaa agctgagaaa aaaggtaatg tacaggtaac aaataaaacc gagaaaacaa ctaaagttga taaaaataat aaagtaccga
5501 aaaaaagaag aacgcaaaaa tcaaaataa . 5529
| 10 | 20 | 30 | 40 | 50 | 60 | 70 | 80 | 90 | 100

Complete nucleotide sequence of the 5529 base-pair (bp) lsa-3 gene. Bolded is a 168 bp intron; underlined are the 3 repeat regions R1, R2 and R3.

PROTEIN: full sequence

1 MTNSNYKSNN KTYNENNNEQ ITTIFNRTNM NPIKKCHMRE KINKYFFLIK ILTCTILIWA VQYDNNSDIN KSWKKNTYVD
81 KKLNKLFNRS LGESQVNGEL ASEEVKEKIL DLLEEGNTLT ESVDDNKNLE EAEDIKENIL LSNIEEPKEN IIDNLLNNIG
161 QNSEKQESVS ENVQVSDELF NELLNSVDVN GEVKENILEE SQVNDDIFNS LVKSVQQEQQ HNVEEKVEES VEENDEESVE
241 ENVEENVEEN DDGSVASSVE ESIASSVDES IDSSIEENVA PTVEEIVAPS VVESVAPSVE ESVEENVEES VAENVEESVA
321 ENVEESVAEN VEESVAENVE EIVAPTVEEI VAPTVEEIVA PSVVESVAPS VEESVEENVE ESVAENVEES VAENVEESVA
401 ENVEESVAEN VEESVAENVE EIVAPTVEEI VAPTVEEIVA PSVVESVAPS VEESVEENVE ESVAENVEES VAENVEESVA
481 ENVEESVAEN VEESVAENVE ESVAENVEES VAENVEEIVA PTVEEIVAPT VEEIVAPSVV ESVAPSVEES VEENVEESVA
561 ENVEESVAEN VEESVAENVE ESVAENVEEI VAPTVEEIVA PTVEEIVAPS VVESVAPSVE ESVEENVEES VAENVEESVA
641 ENVEESVAEN VEEIVAPTVE EIVAPTVEEI VAPSVVESVA PSVEESVEEN VEESVAENVE ESVAENVEES VAENVEESVA
721 ENVEEIVAPT VEEIVAPTVE EIVAPSVVES VAPSVEESVE ENVEESVAEN VEESVAENVE ESVAENVEES VAPTVEEIVA
801 PSVEESVAPS VEESVAENVA TNLSDNLLSN LLGGIETEEI KDSILNEIEE VKENVVTTIL ENVEETTAES VTTFSNILEE
881 IQENTITNDT IEEKLEELHE NVLSAALENT QSEEEKKEVI DVIEEVKEEV ATTLIETVEQ AEEKSANTIT EIFENLEENA
961 VESNENVAEN LEKLNETVFN TVLDKVEETV EISGESLENN EMDKAFFSEI FDNVKGIQEN LLTGMFRSIE TSIVIQSEEK
1041 VDLNENVVSS ILDNIENMKE GLLNKLENIS STEGVQETVT EHVEQNVYVD VDVPAMKDQF LGILNEAGGL KEMFFNLEDV
1121 FKSESDVITV EEIKDEPVQK EVEKETVSII EEMEENIVDV LEEEKEDLTD KMIDAVEESI EISSDSKEET ESIKDKEKDV
1201 SLVVEEVQDN DMDESVEKVL ELKNMEEELM KDAVEINDIT SKLIEETQEL NEVEADLIKD MEKLKELEKA LSEDSKEIID
1281 AKDDTLEKVI EEEHDITTTL DEVVELKDVE EDKIEKVSDL KDLEEDILKE VKEIKELESE ILEDYKELKT IETDILEEKK
1361 EIEKDHFEKF EEEAEEIKDL EADILKEVSS LEVEEEKKLE EVHELKEEVE HIISGDAHIK GLEEDDLEEV DDLKGSILDM
1441 LKGDMELGDM DKESLEDVTT KLGERVESLK DVLSSALGMD EEQMKTRKKA QRPKLEEVLL KEEVKEEPKK KITKKKVRFD
1521 IKDKEPKDEI VEVEMKDEDI EEDVEEDIEE DIEEDKVEDI DEDIDEDIGE DKDEVIDLIV QKEKRIEKVK AKKKKLEKKV
1601 EEGVSGLKKH VDEVMKYVQK IDKEVDKEVS KALESKNDVT NVLKQNQDFF SKVKNFVKKY KVFAAPFISA VAAFASYVVG
1681 FFTFSLFSSC VTIASSTYLL SKVDKTINKN KERPFYSFVF DIFKNLKHYL QQMKEKFSKE KNNNVIEVTN KAEKKGNVQV
1761 TNKTEKTTKV DKNNKVPKKR RTQKSKZ 1786

Complete peptide sequence of the 1786 amino-acid (aa) LSA-3 protein. Bolded are 3 potential start sites; underlined are the 3 repeat regions R1, R2 and R3.

T9/96 PARASITE CLONE Accession Nber AJ007011

Nucleotidic sequence

1' agtgatgaac tttttaatga attattaaat agtgtagatg ttaatggaga agtaaaagaa aatattttgg aggaaagtca
81' agttaatgac gatattttta atagtttagt aaaaagtgtt caacaagaac aacaacacaa tgttgaagaa aaagttgaag
161' aaagtgtaga agaaaatgac gaagaaagtg tagaagaaaa tgtagaagaa aatgtagaag aaaatgacga cggaagtgta
241' gcctcaagtg ttgaagaaag tatagcttca agtgttgatg aaagtataga ttcaagtatt gaagaaaatg tagctccaac
321' tgttgaagaa atcgtagctc caactgttga agaaattgta gctccaagtg ttgtagaaag tgtggctcca agtgttgaag
401' aaagtgtagc tccaagtgtt gaagaaagtg tagctgaaaa tgttgaagaa agtgtagctg aaaatgttga agaaatcgta
481' gctccaagtg ttgaagaaag tgtagctgaa aatgttgaag aaagtgtagc tgaaaatgtt gaagaaagtg tagctgaaaa
561' tgttgaagaa agtgtagctg aaaatgttga agaaagtgta gctgaaaatg ttgaagaaat cgtagctcca actgttgaag
641' aaagtgtagc tccaactgtt gaagaaattg tagctccaac tgttgaagaa agtgtagctc caactgttga agaaattgta
721' gttccaagtg ttgaagaaag tgtagctcca agtgttgaag aaagtgtagc tgaaaatgtt gaagaaagtg tagctgaaaa
801' tgttgaagaa agtgtagctg aaaatgttga agaaagtgta gctgaaaatg ttgaagaaag tgtagctgaa aatgttgaag
881' aaatcgtagc tccaagtgtt gaagaaatcg tagctccaac tgttgaagaa agtgttgctg aaaacgttgc aacaaattta
961' tcagacaatc ttttaagtaa tttattaggt ggtatcgaaa ctgaggaaat aaaggacagt atattaaatg agatagaaga
1041' agtaaaagaa aatgtagtca ccacaatact agaaaaagta gaagaaacta cagctgaaag tgtaactact tttagtaata
1121' tattagagga gatacaagaa aatactatta ctaatgatac tatagaggaa aaattagaag aactccacga aaatgtatta
1201' agtgccgctt tagaaaatac ccaaagtgaa gaggaaaaga aagaagtaat agatgtaatt gaagaagtaa aagaagaggt
1281' cgctaccact ttaatagaaa ctgtggaaca ggcagaagaa gagagcgaaa gtacaattac ggaaatattt gaaaatttag
1361' aagaaaatgc agtagaaagt aatgaaaaag ttgcagagaa tttagagaaa ttaaacgaaa ctgtatttaa tactgtatta
1441' gataaagtag aggaaacagt agaaattagc ggagaaagtt tagaaaacaa tgaaatggat aaagcatttt ttagtgaaat
1521' atttgataat gtaaaaggaa tacaagaaaa tttattaaca ggtatgtttc gaagtataga aaccagtata gtaatccaat
1601' cagaagaaaa ggttgatttg aatgaaaatg tggttagttc gattttagat aatatagaaa atatgaaaga aggtttatta
1681' aataaattag aaaatatttc aagtactgaa gg 1712'

Partial nucleotide sequence of the lsa-3 gene in the Thai parasite clone T9/96. Bolded is the sequence of insert DG729. Insert DG679, the largest among the LSA-3 insert family (see text of the present article and Guérin-Marchand et al., 1987), spans from nucl. 32' to nucl. 1712'. Underlined are the adjacent repeat regions R1 and R2. Position 1' corresponds to nucl. 694 in the original K1 sequence.

Peptidic sequence

1' SDELFNELLN SVDVNGEVKE NILEESQVND DIFNSLVKSV QQEQQHNVEE KVEESVEEND EESVEENVEE NVEENDDGSV
81' ASSVEESIAS SVDESIDSSI EENVAPTVEE IVAPTVEEIV APSVVESVAP SVEESVAPSV EESVAENVEE SVAENVEEIV
161' APSVEESVAE NVEESVAENV EESVAENVEE SVAENVEESV AENVEEIVAP TVEESVAPTV EEIVAPTVEE SVAPTVEEIV
241' VPSVEESVAP SVEESVAENV EESVAENVEE SVAENVEESV AENVEESVAE NVEEIVAPSV EEIVAPTVEE SVAENVATNL
321' SDNLLSNLLG GIETEEIKDS ILNEIEEVKE NVVTTILEKV EETTAESVTT FSNILEEIQE NTITNDTIEE KLEELHENVL
401' SAALENTQSE EEKKEVIDVI EEVKEEVATT LIETVEQAEE ESESTITEIF ENLEENAVES NEKVAENLEK LNETVFNTVL
481' DKVEETVEIS GESLENNEMD KAFFSEIFDN VKGIQENLLT GMFRSIETSI VIQSEEKVDL NENVVSSILD NIENMKEGLL
561' NKLENISSTE 570'

Partial peptide sequence of the LSA-3 protein in the Thai parasite clone T9/96. Bolded is the sequence of insert DG729. Insert DG679, the largest among the LSA-3 insert family (see text of the present article and Guérin-Marchand et al., 1987), spans from aa 12' to aa 570'. Underlined are the 2 adjacent repeat regions R1 and R2. Position 1' corresponds to aa 176 in the original K1 sequence.

Note on LSA-3 sequence in parasite 3D7

The lsa-3 gene sequence in parasite clone 3D7 (derived from strain NF54 used in the present article for chimpanzee challenges) is found in the complete sequence of P. falciparum Chromosome 2 (Gardner et al., 1998) where it was annotated as resa-h3 (Accession Number AE001424).

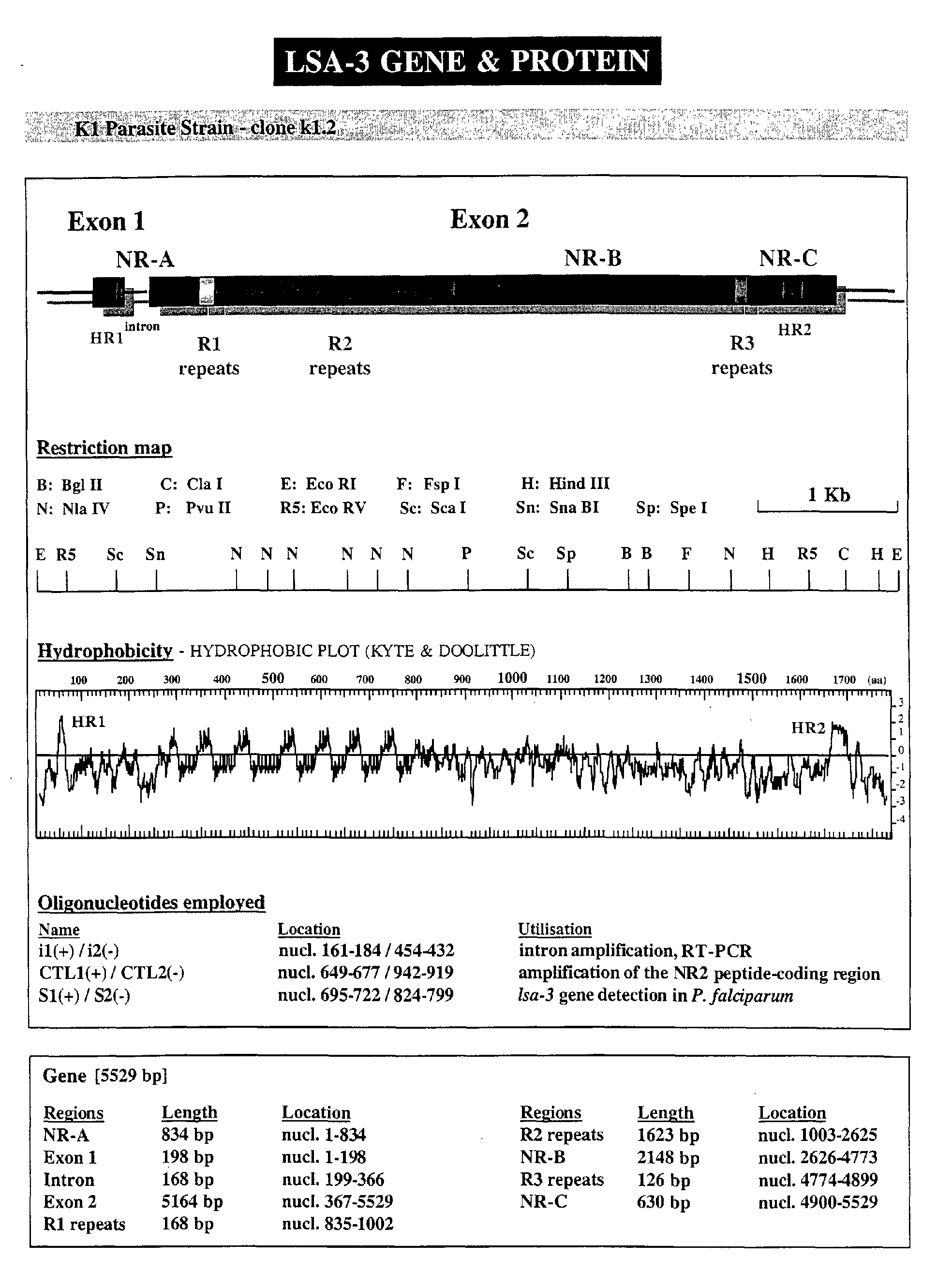
LSA-3 GENE & PROTEIN

K1 Parasite Strain - clone k1.2

Exon 1
Exon 2
NR-A
NR-B
NR-C
intron
HR1
R1
repeats
R2
repeats
R3
repeats
HR2

Restriction map

B: Bgl II | C: Cla I | E: Eco RI | F: Fsp I | H: Hind III
N: Nla IV | P: Pvu II | R5: Eco RV | Sc: Sca I | Sn: Sna BI | Sp: Spe I
1 Kb

E R5 Sc Sn N N N N N N P Sc Sp B B F N H R5 C H E

Hydrophobicity - HYDROPHOBIC PLOT (KYTE & DOOLITTLE)

100 200 300 400 500 600 700 800 900 1000 1100 1200 1300 1400 1500 1600 1700 (aa)
HR1
HR2
3
2
1
0
-1
-2
-3
-4

Oligonucleotides employed

Name | Location | Utilisation
i1(+) / i2(-) | nucl. 161-184 / 454-432 | intron amplification, RT-PCR
CTL1(+) / CTL2(-) | nucl. 649-677 / 942-919 | amplification of the NR2 peptide-coding region
S1(+) / S2(-) | nucl. 695-722 / 824-799 | lsa-3 gene detection in P. falciparum

Gene [5529 bp]

Regions | Length | Location | Regions | Length | Location
NR-A | 834 bp | nucl. 1-834 | R2 repeats | 1623 bp | nucl. 1003-2625
Exon 1 | 198 bp | nucl. 1-198 | NR-B | 2148 bp | nucl. 2626-4773
Intron | 168 bp | nucl. 199-366 | R3 repeats | 126 bp | nucl. 4774-4899
Exon 2 | 5164 bp | nucl. 367-5529 | NR-C | 630 bp | nucl. 4900-5529
R1 repeats | 168 bp | nucl. 835-1002

Protein [786 amino acids - Predicted MW : 200 kDa]

Regions | Length | Location |
NR-A | 278 aa | aa 1-278 | Non-repeated region A
HR1 | 18 aa | aa 46-63 | Hydrophobic region 1: putative signal peptide
R1 | 56 aa | aa 223-278 | Conserved repeat region
R2 | 541 aa | aa 279-819 | Polymorphic repeat region
NR-B | 716 aa | aa 820-1535 | Non-repeated region B
R3 | 42 aa | aa 1536-1577 | Conserved repeat region
NR-C | 210 aa | aa 1578-1786 | Non-repeated region C
HR2 | 33 aa | aa 1662-1694 | Hydrophobic region 2: putative
 | | | transmembrane domain

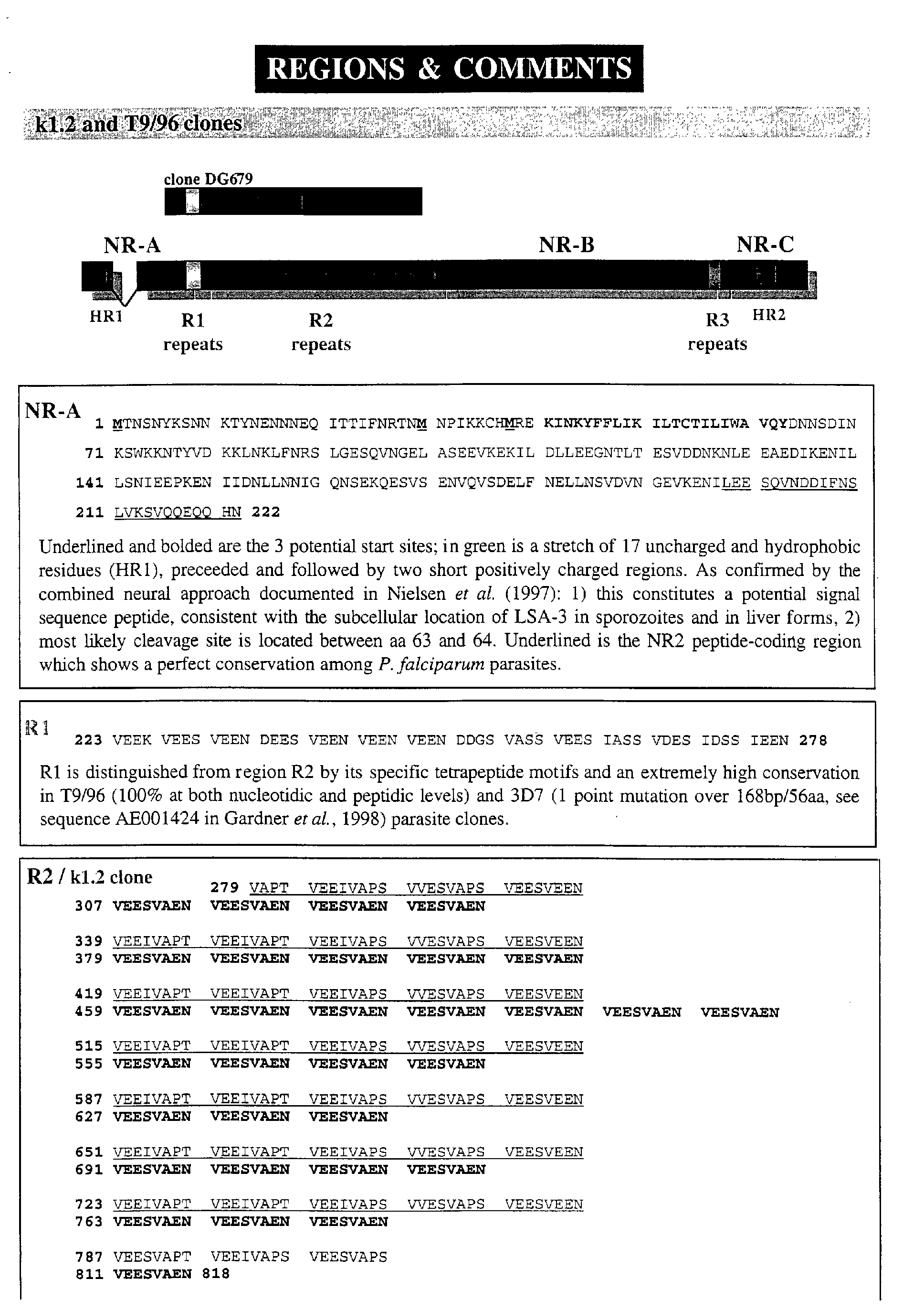
REGIONS & COMMENTS

k1.2 and T9/96 clones

clone DG679


NR-A | NR-B | NR-C

HR1 | R1 repeats | R2 repeats | R3 repeats | HR2

NR-A
1 MTNSNYKSNN KTYNENNNEQ ITTIFNRTNM NPIKKCHMRE KINKYFFLIK ILTCTILIWA VQYDNNSDIN
71 KSWKKNTYVD KKLNKLFNRS LGESQVNGEL ASEEVKEKIL DLLEEGNTLT ESVDDNKNLE EAEDIKENIL
141 LSNIEEPKEN IIDNLLNNIG QNSEKQESVS ENVQVSDELF NELLNSVDVN GEVKENILEE SQVNDDIFNS
211 LVKSVQQEQQ HN 222

Underlined and bolded are the 3 potential start sites; in green is a stretch of 17 uncharged and hydrophobic residues (HR1), preceeded and followed by two short positively charged regions. As confirmed by the combined neural approach documented in Nielsen et al. (1997): 1) this constitutes a potential signal sequence peptide, consistent with the subcellular location of LSA-3 in sporozoites and in liver forms, 2) most likely cleavage site is located between aa 63 and 64. Underlined is the NR2 peptide-coding region which shows a perfect conservation among P. falciparum parasites.

R1
223 VEEK VEES VEEN DEES VEEN VEEN VEEN DDGS VASS VEES IASS VDES IDSS IEEN 278

R1 is distinguished from region R2 by its specific tetrapeptide motifs and an extremely high conservation in T9/96 (100% at both nucleotidic and peptidic levels) and 3D7 (1 point mutation over 168bp/56aa, see sequence AE001424 in Gardner et al., 1998) parasite clones.

R2 / k1.2 clone
279 VAPT VEEIVAPS VVESVAPS VEESVEEN
307 VEESVAEN VEESVAEN VEESVAEN VEESVAEN

339 VEEIVAPT VEEIVAPT VEEIVAPS VVESVAPS VEESVEEN
379 VEESVAEN VEESVAEN VEESVAEN VEESVAEN VEESVAEN

419 VEEIVAPT VEEIVAPT VEEIVAPS VVESVAPS VEESVEEN
459 VEESVAEN VEESVAEN VEESVAEN VEESVAEN VEESVAEN VEESVAEN VEESVAEN

515 VEEIVAPT VEEIVAPT VEEIVAPS VVESVAPS VEESVEEN
555 VEESVAEN VEESVAEN VEESVAEN VEESVAEN

587 VEEIVAPT VEEIVAPT VEEIVAPS VVESVAPS VEESVEEN
627 VEESVAEN VEESVAEN VEESVAEN

651 VEEIVAPT VEEIVAPT VEEIVAPS VVESVAPS VEESVEEN
691 VEESVAEN VEESVAEN VEESVAEN VEESVAEN

723 VEEIVAPT VEEIVAPT VEEIVAPS VVESVAPS VEESVEEN
763 VEESVAEN VEESVAEN VEESVAEN

787 VEESVAPT VEEIVAPS VEESVAPS
811 VEESVAEN 818

R2 / T9/96 clone
104' VAPT VEEIVAPT VEEIVAPS VVESVAPS VEESVAPS
140' VEESVAEN VEESVAEN
156' VEEIVAPS
164' VEESVAEN VEESVAEN VEESVAEN VEESVAEN VEESVAEN
204' VEEIVAPT VEESVAPT VEEIVAPT VEESVAPT VEEIVVPS VEESVAPS
252' VEESVAEN VEESVAEN VEESVAEN VEESVAEN VEESVAEN
292' VEEIVAPS VEEIVAPT
308' VEESVAEN 315'

Bolded are stretches of tandemly repeated and conserved octapeptides VEESVAEN which can vary in number, from 2 to 7 in both strains. Underlined are the highly conserved 40 aa repeated blocks which separate these stretches in strain K1. In clone T9/96 , no particular organization is observed in R2. This region is nevertheless composed of similar and conserved tetrapeptides compared to strain K1, except one variant VVPS which is specific for T9/96.

NR-B
819 VA TNLSDNLLSN LLGGIETEEI KDSILNEIEE VKENVVTTIL ENVEETTAES VTTFSNILEE
881 IQENTITNDT IEEKLEELHE NVLSAALENT QSEEEKKEVI DVIEEVKEEV ATTLIETVEQ AEEKSANTIT
951 EIFENLEENA VESNENVAEN LEKLNETVFN TVLDKVEETV EISGESLENN EMDKAFFSEI FDNVKGIQEN
1021 LLTGMFRSIE TSIVIQSEEK VDLNENVVSS ILDNIENMKE GLLNKLENIS STEGVQETVT EHVEQNVYVD
1091 VDVPAMKDQF LGILNEAGGL KEMFFNLEDV FKSESDVITV EEIKDEPVQK EVEKETVSII EEMEENIVDV
1161 LEEEKEDLTD KMIDAVEESI EISSDSKEET ESIKDKEKDV SLVVEEVQDN DMDESVEKVL ELKNMEEELM
1231 KDAVEINDIT SKLIEETQEL NEVEADLIKD MEKLKELEKA LSEDSKEIID AKDDTLEKVI EEEHDITTTL
1301 DEVVELKDVE EDKIEKVSDL KDLEEDILKE VKEIKELESE ILEDYKELKT IETDILEEKK EIEKDHFEKF
1371 EEEAEEIKDL EADILKEVSS LEVEEEKKLE EVHELKEEVE HIISGDAHIK GLEEDDLEEV DDLKGSILDM
1441 LKGDMELGDM DKESLEDVTT KLGERVESLK DVLSSALGMD EEQMKTRKKA QRPKLEEVLL KEEVKEEPKK
1511 KITKKKVRFD IKDKEPKDEIVEVEM 1535

Underlined is the partial NR-B region of insert DG679 (parasite clone T9/96) which shows a high degree of conservation with K1 sequences and contains only 6 bp substitutions leading to 5 aa mutations (bolded). Shaded is the highly conserved HLA-B53 restricted epitope la90 identified by Aidoo et al. (2000).

R3
1536 KDED IEED VEED IEED IEED KVED IDED IDED IGED KDEV ID 1577

The same regular spacing of the hydrophobic isoleucine and valine residues is observed in region R3 which is predicted, according to its HCP analysis (not shown), to adopt an α-helical conformation and is preceded by a cluster of helix-breakers (proline) alternating with ß-sheet segments. This region also shows a high degree of conservation with LSA-3 sequences in clone 3D7 (see sequence AE001424 in Gardner et al., 1998) and in isolates from various geographical origins (Daubersies, P. et al., in preparation).

NR-C
1578 LIV QKEKRIEKVK AKKKKLEKKV EEGVSGLKKH VDEVMKYVQK IDKEVDKEVS KALESKNDVT
1641 NVLKQNQDFF SKVKNFVKKY KVFAAPFISA VAAFASYVVG FFTFSLFSSC VTIASSTYLL SKVDKTINKN
1711 KERPFYSFVF DIFKNLKHYL QQMKEKFSKE KNNNVIEVTN KAEKKGNVQV TNKTEKTTKV DKNNKVPKKR
1781 RTQKSKZ 1786

Bolded (and in green) is a second hydrophobic region (HR2) which could constitute a transmembrane domain, consistent with the subcellular location of the antigen in sporozoites and in liver forms.

CONSERVATION

Conservation of the gene

LSA-3 gene and protein detected in 100 % of P. falciparum parasites by:

• PCR ANALYSIS performed with S1(+) / S2(-) primers on:

- 70 isolates from Ivory Coast, Madagascar, Myanmar, Brazil and Columbia
- 12 Thai sporozoite strains
- 6 laboratory strains or clones (K1, T9/96, NF54, Palo Alto, 150, 3D7)
- 23 Senegalese isolates - Data published in Bottius et al. (1996) [where clone DG157 is a member
of the LSA-3 clone family and encodes for a part of region NR-A]

The expected 130 bp amplification product was found in the 111 samples

•IFAT performed with anti-NR2 peptide and anti-GST-PC antibodies (mouse and chimpanzee sera) on:

- 30 Thai sporozoite strains
- 2 infected liver sections: one from a Cebus (day 5 post-challenge) and one from a chimpanzee (day 6
post-challenge)

Detection in the 32 samples
100 % of positive parasites in each assay

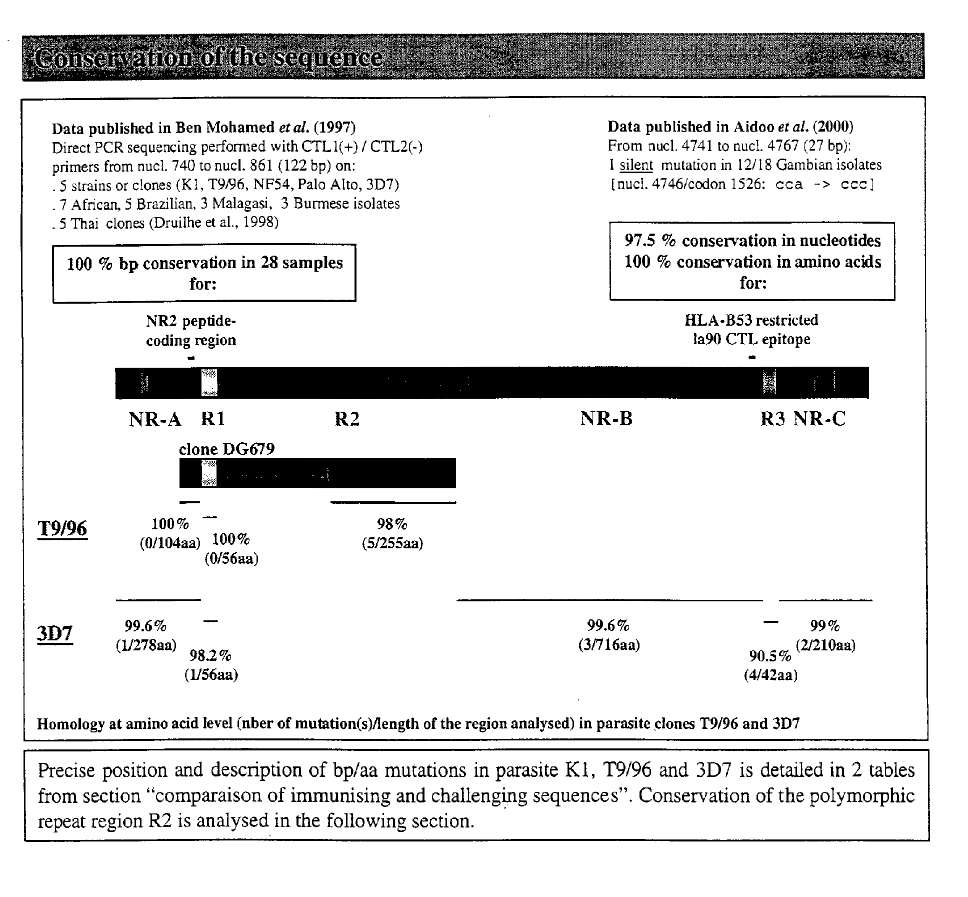
Conservation of the sequence

Data published in Ben Mohamed et al. (1997)
Direct PCR sequencing performed with CTL1(+) / CTL2(-)
primers from nucl. 740 to nucl. 861 (122 bp) on:
. 5 strains or clones (K1, T9/96, NF54, Palo Alto, 3D7)
. 7 African, 5 Brazilian, 3 Malagasi, 3 Burmese isolates
. 5 Thai clones (Druilhe et al., 1998)

100 % bp conservation in 28 samples
for:

Data published in Aidoo et al. (2000)
From nucl. 4741 to nucl. 4767 (27 bp):
1 silent mutation in 12/18 Gambian isolates
[nucl. 4746/codon 1526: cca -> ccc]

97.5 % conservation in nucleotides
100 % conservation in amino acids
for:

NR2 peptide-
coding region

HLA-B53 restricted
la90 CTL epitope


NR-A R1 R2 NR-B R3 NR-C

clone DG679


T9/96

100%
(0/104aa)

100%
(0/56aa)

98%
(5/255aa)

3D7

99.6%
(1/278aa)

98.2%
(1/56aa)

99.6%
(3/716aa)

90.5%
(4/42aa)

99%
(2/210aa)

Homology at amino acid level (nber of mutation(s)/length of the region analysed) in parasite clones T9/96 and 3D7

Precise position and description of bp/aa mutations in parasite K1, T9/96 and 3D7 is detailed in 2 tables
from section "comparaison of immunising and challenging sequences". Conservation of the polymorphic
repeat region R2 is analysed in the following section.

Conservation of the repeat region R2

Conservation of R2 motif sequences

MOTIFS | P. FALCIPARUM LINES
PEPTIDIC | NUCLEOTIDIC | K1 | T9/96 | 3D7
VAEN | gta gct gaa aat | 30/31 | 12/13 | 9/10
 | --t --- --- --c | 1/31 | 1/13 | 1/10
VAPS | gta gct cca agt | 9/16 | 5/6 | 16/17
 | --g --- --- --- | 7/16 | 1/6 | 1/17
VAPT | gta gct cca act | 14/14 | 7/7 | 7/9
 | --- --- --- --a | - | - | 2/9
VEES | gtt gaa gaa agt | 42/42 | 17/17 | 15/15
VEEI | gtt gaa gaa atc | 13/20 | 5/8 | 16/22
 | --- --- --- --t | 7/20 | 3/8 | 6/22
VEEN | gta gaa gaa aat | 11/11 | - | 1/1
VVES | gtt gta gaa agt | 7/7 | - | -
 | --c --- --- --- | - | - | 1/1
VVPS | gta gtt cca agt | - | 1/1 | -
VVPT | gta gtt cca act | - | - | 2/2

Peptide and nucleotide sequence comparison of R2 tetrapeptidic motifs between K1, T9/96 and 3D7
parasites. Although the organization of these tetrapeptide motifs varies within R2 (see section "regions &
comments" for K1, and T9/96 and see sequence AE001424 in Gardner et al. (1998) for 3D7),
conservation of their sequences remains extremly high (e.g. only 3 strain specific tetrapeptides
(VVPS, VVPT) among a total of 231 motifs and no single nucleotide mutation in the 74 VEES, 21 VAPT,
12 VEEN motifs.

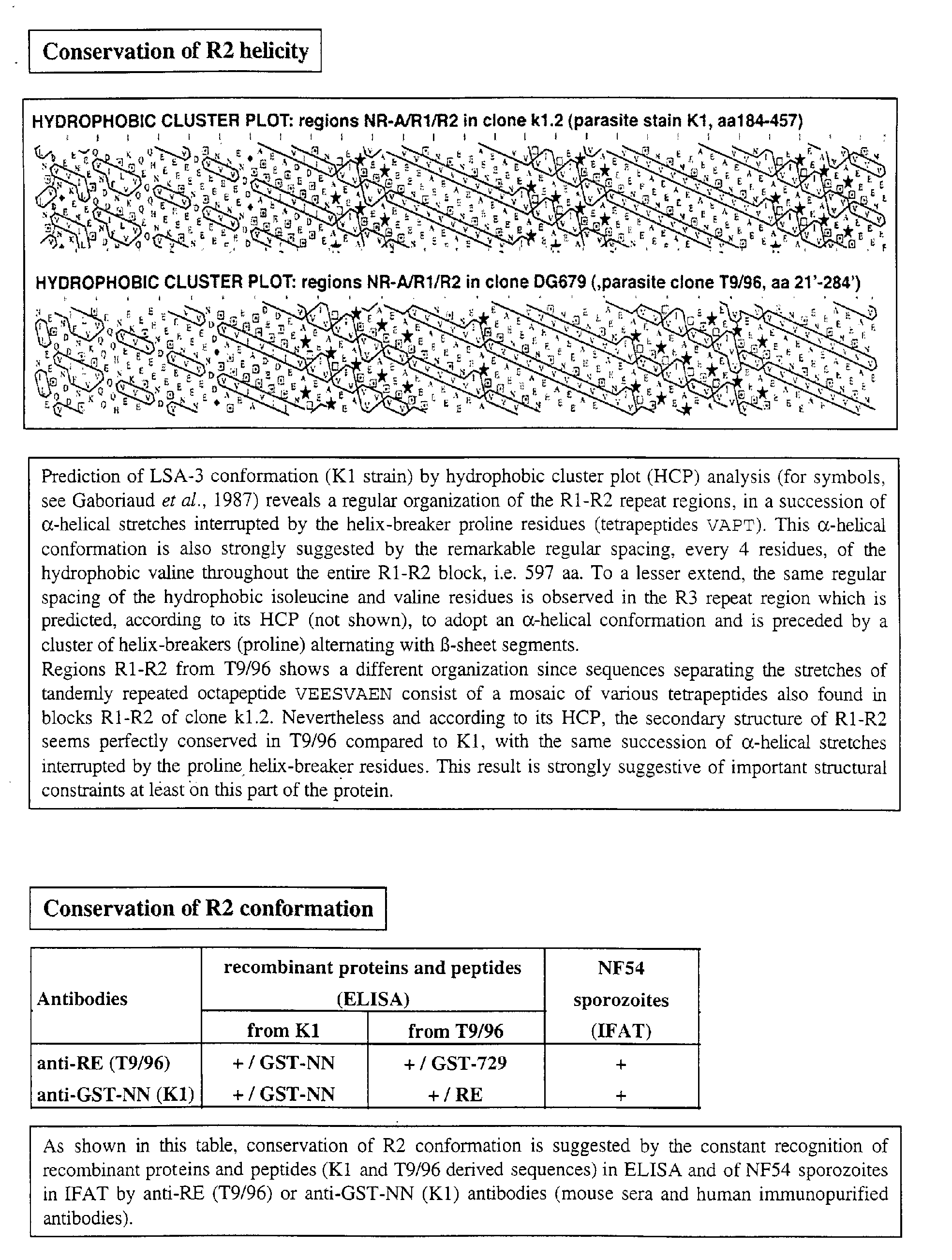
Conservation of R2 helicity

HYDROPHOBIC CLUSTER PLOT: regions NR-A/R1/R2 in clone k1.2 (parasite stain K1, aa184-457)
HYDROPHOBIC CLUSTER PLOT: regions NR-A/R1/R2 in clone DG679 (,parasite clone T9/96, aa 21'-284')

Prediction of LSA-3 conformation (K1 strain) by hydrophobic cluster plot (HCP) analysis (for symbols,
see Gaboriaud et al., 1987) reveals a regular organization of the R1-R2 repeat regions, in a succession of
α-helical stretches interrupted by the helix-breaker proline residues (tetrapeptides VAPT). This α-helical
conformation is also strongly suggested by the remarkable regular spacing, every 4 residues, of the
hydrophobic valine throughout the entire R1-R2 block, i.e. 597 aa. To a lesser extend, the same regular
spacing of the hydrophobic isoleucine and valine residues is observed in the R3 repeat region which is
predicted, according to its HCP (not shown), to adopt an α-helical conformation and is preceded by a
cluster of helix-breakers (proline) alternating with ß-sheet segments.
Regions R1-R2 from T9/96 shows a different organization since sequences separating the stretches of
tandemly repeated octapeptide VEESVAEN consist of a mosaic of various tetrapeptides also found in
blocks R1-R2 of clone k1.2. Nevertheless and according to its HCP, the secondary structure of R1-R2
seems perfectly conserved in T9/96 compared to K1, with the same succession of α-helical stretches
interrupted by the proline helix-breaker residues. This result is strongly suggestive of important structural
constraints at least on this part of the protein.

Conservation of R2 conformation

Antibodies | recombinant proteins and peptides (ELISA) | | NF54 sporozoites (IFAT)
| from K1 | from T9/96 |
anti-RE (T9/96) | + / GST-NN | + / GST-729 | +
anti-GST-NN (K1) | + / GST-NN | + / RE | +

As shown in this table, conservation of R2 conformation is suggested by the constant recognition of
recombinant proteins and peptides (K1 and T9/96 derived sequences) in ELISA and of NF54 sporozoites
in IFAT by anti-RE (T9/96) or anti-GST-NN (K1) antibodies (mouse sera and human immunopurified
antibodies).

Comparison between immunising and challenging sequences

Mutations identified and localisation

LSA-3 Regions¹ | Clones² | Mutated nucleotide³ | Mutated codon³ | Original K1 sequence⁴ | Mutated sequence⁴
NR-A (1-834) | 3D7 | 191 | 64 | gat (D) | gct (A)
R1 (835-1002) | 3D7 | 926 | 253 | gga (G) | gct (E)
NR-B (2626-4773) | T9/96 | 2754 | 862 | aac (N) | aaa (K)
| T9/96 | 2796 | 876 | aac (N) | aat sil.
| 3D7 + T9/96 | 2998 | 944 | aag (K) | gag (E)
| T9/96 | 3005 | 946 | gca (A) | gag (E)
| 3D7 + T9/96 | 3008 | 947 | aat (N) | agt (S)
| T9/96 | 3066 | 966 | aat (N) | aaa (K)
| 3D7 | 3972 | 1268 | gaa (E) | gag sil.
| 3D7 | 4546 | 1460 | aca (T) | gca (A)
| 3D7 | 4650 | 1494 | aag (K) | aaa sil.
R3 (4774-4899) | 3D7 | 4791 | 1541 | gaa (E) | gat (D)
| 3D7 | 4798 | 1544 | gta (V) | ata (I)
| 3D7 | 4810 | 1548 | ata (I) | gta (V)
| 3D7 | 4870-71 | 1567-68 | - | 12 bp ins.⁵
NR-C (4900-5529) | 3D7 | 4940 | 1591 | gcg (A) | gag (E)
| 3D7 | 5508 | 1780 | aga (R) | agt (S)

Position in the reference lsa-3 gene (strain K1) and description of the mutations identified in
parasites clones T9/96 and 3D7 (which was originally cloned from strain NF54 and is
considered here as representative of NF54 for complete comparison purposes). As reported in
section "conservation of the sequence", NR2 peptide-coding region of the NF54 strain used
for the chimpanzee challenges was found 100 % homologuous to K1 sequence.
1. Comments on region R2 from K1, T9/96 and 3D7 parasites are given in the preceeding
section. Numbers in brackets define first and last nucleotides of the corresponding region in
strain K1. 2. 3D7 sequences analysed here cover the entire gene and were defined by
compiling data from 3 different sources: 1) construct VR2555 which contains a PCR-
amplified truncated lsa-3 gene (nucl. 432-5095; P. Daubersies, unpublished data), 2)
construct VR2556 which contains a full-length PCR-amplified LSA-3 cDNA (Hoffman S.,
personal communication), 3) lsa-3 gene sequence identified in P. falciparum Chromosome 2
(seq. AE001424 in Gardner et al., 1998). Mutations were considered as such if they were
observed in at least 2 out of 3 sequences. 3. Numbers for mutated nucleotides and codons
correspond to their location in the reference lsa-3 gene and protein respectively (in strain K1).
4. Original and mutated codons are followed in brackets with the corresponding amino acid
(one-letter code). 5. 12 base pair insertion "gaagatatagat", leading to a 4 amino acid
insertion "EDID".

Correspondences and homologies

LSA-3 regions | LSA-3 sequences¹
| in strain K1 | in clone T9/96 | in clone 3D7
| sequenced | immunis.² | sequenced | immunis.³ | sequenced⁴ | challenge⁵
NR-A | length in base pairs | 834 | 60 (CT1) | 316 | 141 (GST-729) | 834 | 60 + 141
| location in gene | 1-834 | 586-645 | 519-834 | 694-834 | 1-834 | 586-645 + 694-834
| length in amino acids | 278 | 20 | 104 | 47 | 278 | 20 + 47
| location in protein | 1-278 | 140-159 | 119-222 | 176-222 | 1-278 | 140-159 + 176-222
| nucleotid. mutation(s) | | | 0 | 0 | 1 | 0
| aa mutation(s) | | | 0 | 0 | 1 | 0
R1 | length in base pairs | 168 | - | 168 | 168 (GST-729) | 168 | 168
| location in gene | 835-1002 | | 835-1002 | 835-1002 | 835-1002 | 835-1002
| length in amino acids | 56 | - | 56 | 56 | 56 | 56
| location in protein | 223-278 | | 223-278 | 223-278 | 223-278 | 223-278
| nucleotid. mutation(s) | | | 0 | 0 | 1 | 1
| aa mutation(s) | | | 0 | 0 | 1 | 1
R2⁶ | length in base pairs | 1623 | 240 (GST-NN) | 636 (full seq.) | 141 (GST-729) | 924 (full seq.) | 924
| location in gene | 1003-2625 | 1269-1509 | | | |
| length in amino acids | 541 | 80 | 212 | 47 | 308 | 308
| location in protein | 279-819 | 369-448 | | | |
NR-B | length in base pairs | 2148 | 2006 (GST-PC) | 764 | - | 2148 | 2009
| location in gene | 2626-4773 | 2769-4773 | 2626-3389 | | 2626-4773 | 2769-4773
| length in amino acids | 716 | 667 | 255 | - | 716 | 667
| location in protein | 820-1535 | 869-1535 | 820-1074 | | 820-1535 | 869-1535
| nucleotid. mutations) | | | 6 | | 5 | 5
| aa mutation(s) | | | 5 | | 3 | 3
R3 | length in base pairs | 126 | 126 (GST-PC) | - | - | 126 | 126
| location in gene | 4774-4899 | 4774-4899 | | | 4774-4899 | 4774-4899
| length in amino acids | 42 | 42 | - | - | 42 | 42
| location in protein | 1536-1577 | 1536-1577 | | | 1536-1577 | 1536-1577
| nucleotid. mutations | | | | | 4 | 4
| aa mutation(s) | | | | | 4 | 4
NR-C | length in base pairs | 630 | 630 (GST-PC) | - | - | 630 | 630
| location in gene | 4900-5529 | 4900-5529 | | | 4900-5529 | 4900-5529
| length in amino acids | 210 | 210 | - | - | 210 | 210
| location in protein | 1578-1786 | 1578-1786 | | | 1578-1786 | 1578-1786
| nucleotid. mutations | | | | | 2 | 2
| aa mutation(s) | | | | | 2 | 2
Non-repeated regions (NR-A, -B, -C) | total length in bp/aa | 3612/1204 | 2695/898 | 1080/360 | | 3612/1204 | 2836/944
| total nber nucl./aa mut. | | | 6/5 | | 8/6 | 7/5
| nucl./aa homology (%) | | | 99.4/98.6 | | 99.8/99.5 | 99.8/99.5
Conserved regions (NR-A,-B,-C, R1,R3) | total length in bp/aa | 3906/1302 | 2821/940 | 1248/416 | | 3906/1302 | 3130/1042
| total nber nucl./aa mut. | | | 6/5 | | 13/11 | 12/10
| nucl./aa homology (%) | | | 99.5/98.8 | | 99.7/99.1 | 99.6/99.0

Definition and comparison of immunising and challenging sequences. As in the preceeding table,
lsa-3 sequence in clone 3D7 (originally cloned from NF54 strain) is considered here as
representative of the actual NF54 strain used for sporozoite challenges.
1: All sequence locations (bp and aa) correspond to the reference numbering in lsa-3 gene and
protein from strain K1. 2: Immunising sequences in strain K1 correspond to peptide CT1 and
recombinant proteins GST-NN and GST-PC. 3: Immunising sequences in clone T9/96 correspond
to peptides NR1, NR2, and RE and recombinant protein GST-729 from which these 3 peptides
were derived. 4: See note (2) in the preceeding table. 5: Challenging sequences are defined as 3D7
sequences corresponding to cumulated immunising sequences from both K1 and T9/96 parasites. 6:
A more detailed analysis of R2 is given in the preceeding section. Due to length polymorphism,
numbering in region R2 is non-relevant in parasites other than K1. Lengths given for T9/96 and
3D7 correspond to their respective fully sequenced region R2.
39

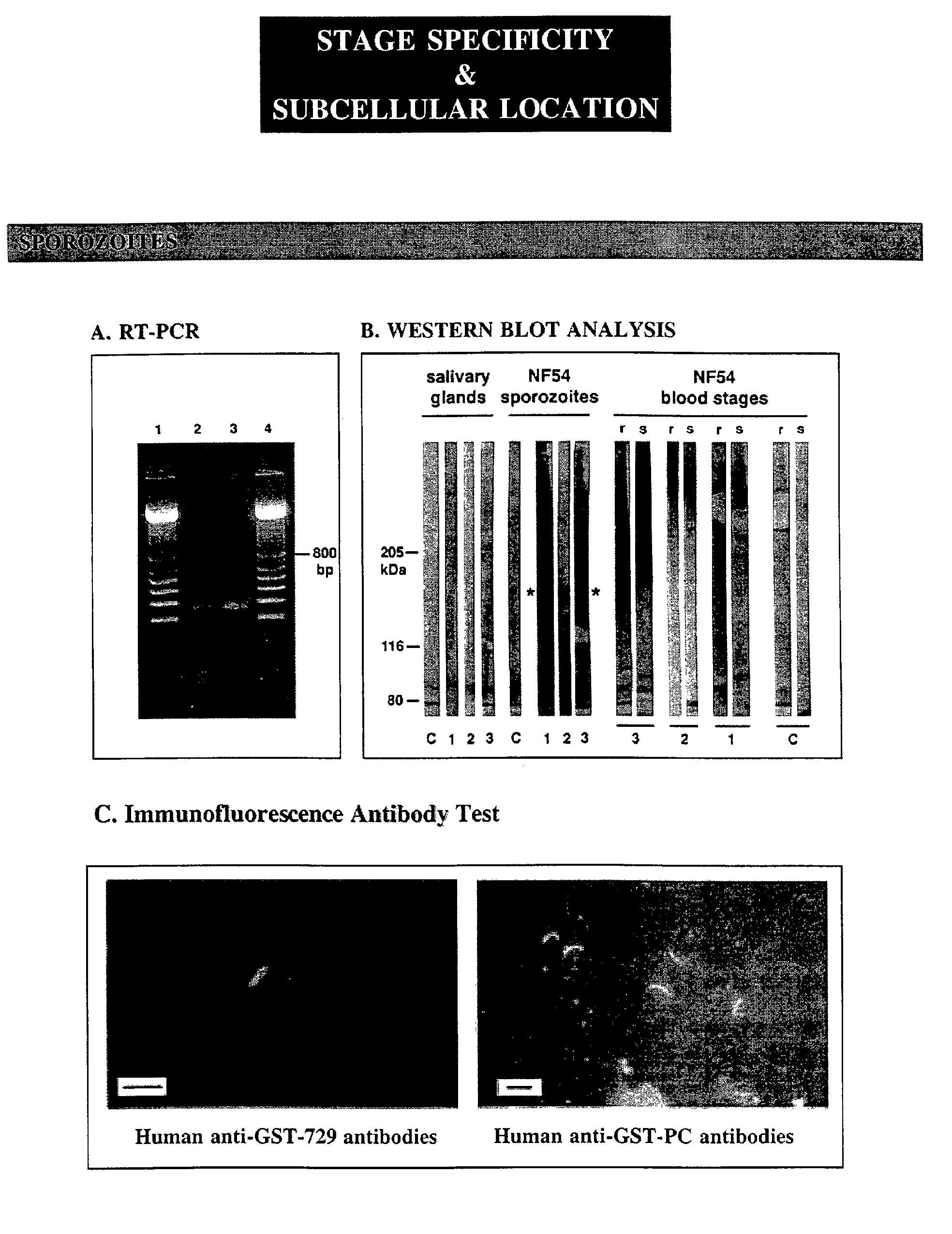
STAGE SPECIFICITY
&
SUBCELLULAR LOCATION

SPOROZOITES

A. RT-PCR

B. WESTERN BLOT ANALYSIS


1 2 3 4

800
bp

salivary
glands

NF54
sporozoites

NF54
blood stages

r s r s r s r s

205
kDa

116

80

*

*

C 1 2 3 C 1 2 3 3 2 1 C

C. Immunofluorescence Antibody Test


Human anti-GST-729 antibodies

Human anti-GST-PC antibodies

A. Due to the difficulties in obtaining an adequate quantity of sporozoite mRNA, Northern blot analysis
could not be performed at this stage and transcription of lsa-3 gene was studied by RT-PCR.
Oligonucleotides i1 (+) and i2 (-), located 3' of exon 1 and 5' of exon 2 respectively, allowed amplification
of the expected 125 bp fragment in NF54 mRNA (lane 2) whereas control DNA (lane 3) and
contaminating DNA (lane 2) gave a 293 bp band. Lanes 1: 100 bp ladder (Amersham). Effective splicing
of the intron were further confirmed by subcloning of the 125 bp fragment and complete sequencing.
B. Western blot analysis of protein extracts from uninfected mosquito salivary glands, NF54 sporozoites
and blood stages (r: rings, s: schizonts) using mouse antisera directed against C) control GST recombinant
protein, 1) GST-PC recombinant protein, 2) oligonucleotides GP5-GP6-GP8-GP11, 3) GST-729
recombinant protein (see Methods). In sporozoites, LSA-3 is visualized as a 175 kDa protein (*), in
agreement with LSA-3 theoretical molecular weight calculated (for NF54 sequence) with the
PEPTIDEMASS program (Wilkins et al., 1997 and http://www.expasy.ch/tools/peptide-mass.html). C.
By IFAT, LSA-3 appears to be located in some areas of the membrane and to distribute over the
cytoplasm of sporozoites. Bars correspond to 10 μm.

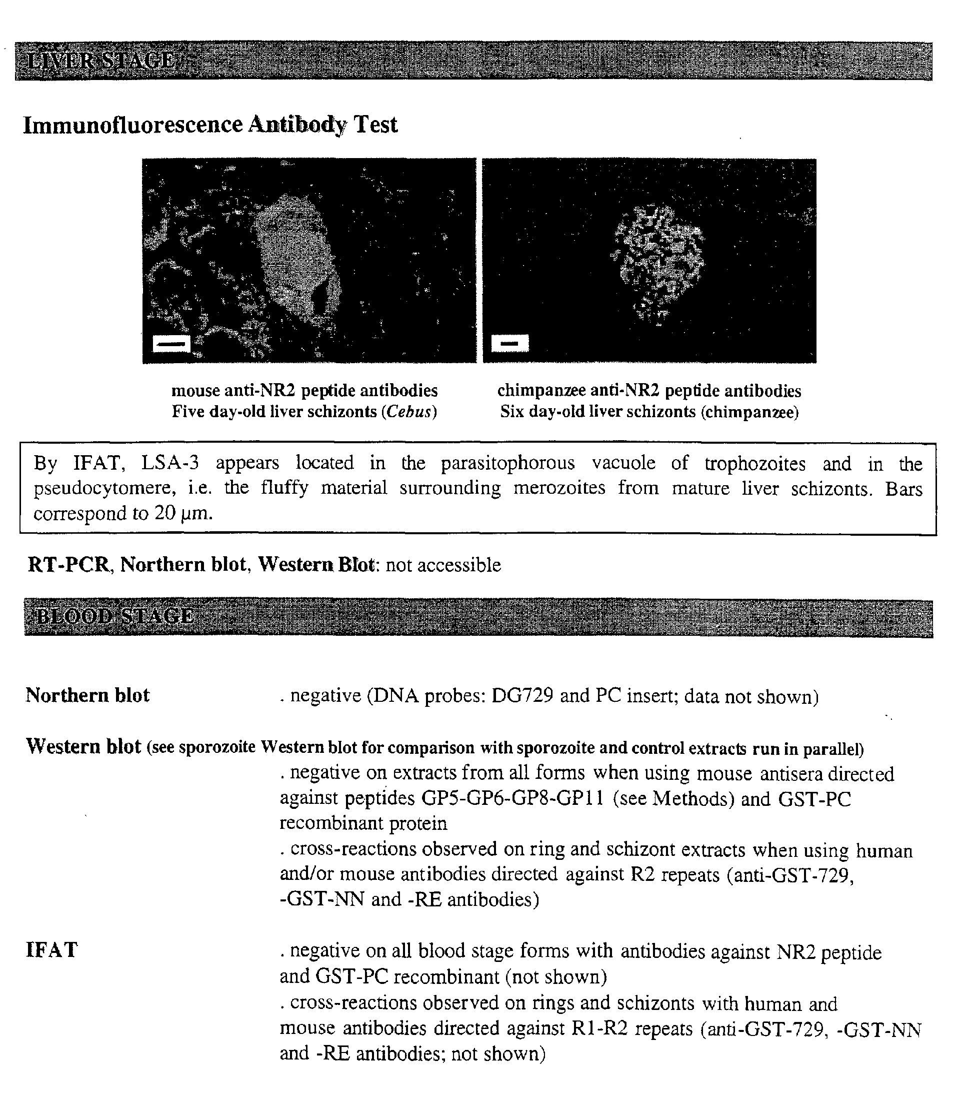
LIVER STAGE

Immunofluorescence Antibody Test



mouse anti-NR2 peptide antibodies
Five day-old liver schizonts (Cebus)
chimpanzee anti-NR2 peptide antibodies
Six day-old liver schizonts (chimpanzee)

By IFAT, LSA-3 appears located in the parasitophorous vacuole of trophozoites and in the
pseudocytomere, i.e. the fluffy material surrounding merozoites from mature liver schizonts. Bars
correspond to 20 μm.

RT-PCR, Northern blot, Western Blot: not accessible

BLOOD STAGE

Northern blot . negative (DNA probes: DG729 and PC insert; data not shown)

Western blot (see sporozoite Western blot for comparison with sporozoite and control extracts run in parallel)
. negative on extracts from all forms when using mouse antisera directed
against peptides GP5-GP6-GP8-GP11 (see Methods) and GST-PC
recombinant protein
. cross-reactions observed on ring and schizont extracts when using human
and/or mouse antibodies directed against R2 repeats (anti-GST-729,
-GST-NN and -RE antibodies)

IFAT . negative on all blood stage forms with antibodies against NR2 peptide
and GST-PC recombinant (not shown)
. cross-reactions observed on rings and schizonts with human and
mouse antibodies directed against R1-R2 repeats (anti-GST-729, -GST-NN
and -RE antibodies; not shown)

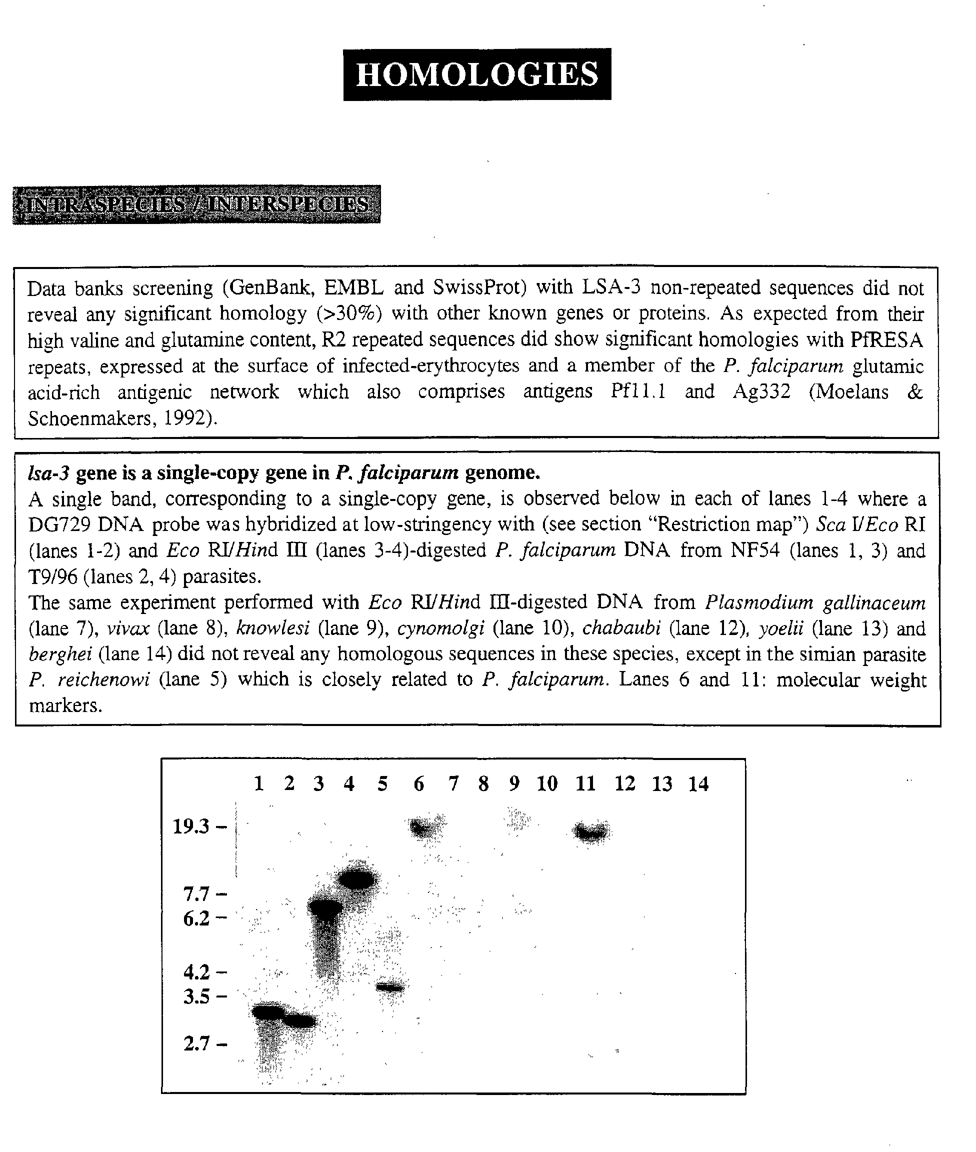
HOMOLOGIES

INTRASPECIES / INTERSPECIES

Data banks screening (GenBank, EMBL and SwissProt) with LSA-3 non-repeated sequences did not
reveal any significant homology (>30%) with other known genes or proteins. As expected from their
high valine and glutamine content, R2 repeated sequences did show significant homologies with PfRESA
repeats, expressed at the surface of infected-erythrocytes and a member of the P. falciparum glutamic
acid-rich antigenic network which also comprises antigens Pf11.1 and Ag332 (Moelans &
Schoenmakers, 1992).

lsa-3 gene is a single-copy gene in P. falciparum genome.
A single band, corresponding to a single-copy gene, is observed below in each of lanes 1-4 where a
DG729 DNA probe was hybridized at low-stringency with (see section "Restriction map") Sca I/Eco RI
(lanes 1-2) and Eco RI/Hind III (lanes 3-4)-digested P. falciparum DNA from NF54 (lanes 1, 3) and
T9/96 (lanes 2, 4) parasites.
The same experiment performed with Eco RI/Hind III-digested DNA from Plasmodium gallinaceum
(lane 7), vivax (lane 8), knowlesi (lane 9), cynomolgi (lane 10), chabaubi (lane 12), yoelii (lane 13) and
berghei (lane 14) did not reveal any homologous sequences in these species, except in the simian parasite
P. reichenowi (lane 5) which is closely related to P. falciparum. Lanes 6 and 11: molecular weight
markers.

1 2 3 4 5 6 7 8 9 10 11 12 13 14
19.3 -
7.7 -
6.2 -
4.2 -
3.5 -
2.7 -

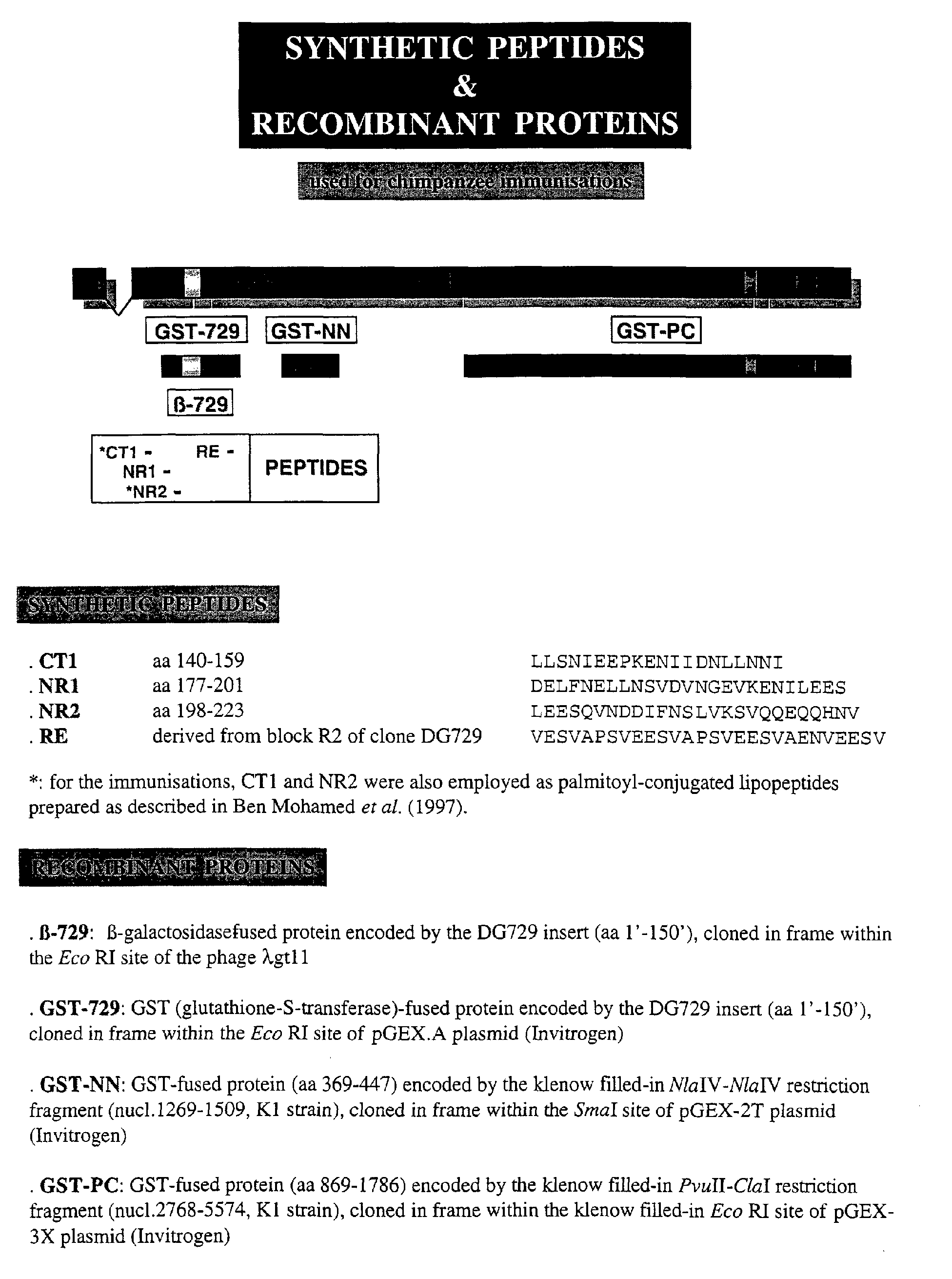
SYNTHETIC PEPTIDES
&
RECOMBINANT PROTEINS

used for chimpanzee immunisations


GST-729 | GST-NN | GST-PC

ß-729

*CT1 - RE -
NR1 -
*NR2 - | PEPTIDES

SYNTHETIC PEPTIDES

. CT1 aa 140-159 LLSNIEEPKENIIDNLLNNI
. NR1 aa 177-201 DELFNELLNSVDVNGEVKENILEES
. NR2 aa 198-223 LEESQVNDDIFNSLVKSVQQEQQHNV
. RE derived from block R2 of clone DG729 VESVAPSVEESVAPSVEESVAENVEESV

*: for the immunisations, CT1 and NR2 were also employed as palmitoyl-conjugated lipopeptides
prepared as described in Ben Mohamed et al. (1997).

RECOMBINANT PROTEINS

. ß-729: ß-galactosidasefused protein encoded by the DG729 insert (aa 1'-150'), cloned in frame within
the Eco RI site of the phage λgt11

. GST-729: GST (glutathione-S-transferase)-fused protein encoded by the DG729 insert (aa 1'-150'),
cloned in frame within the Eco RI site of pGEX.A plasmid (Invitrogen)

. GST-NN: GST-fused protein (aa 369-447) encoded by the klenow filled-in NlaIV-NlaIV restriction
fragment (nucl.1269-1509, K1 strain), cloned in frame within the SmaI site of pGEX-2T plasmid
(Invitrogen)

. GST-PC: GST-fused protein (aa 869-1786) encoded by the klenow filled-in PvuII-ClaI restriction
fragment (nucl.2768-5574, K1 strain), cloned in frame within the klenow filled-in Eco RI site of pGEX-
3X plasmid (Invitrogen)

### METHODS

### 1. Parasites

Blood stages of *P. falciparum* T9/96 clone (Thaithong *et al*., 1984), NF54 (Ponnudurai et *al*., 19881) and K1 (Thaithong and Beale, 1981) strains were cultured as described by Trager and Jensen (1976). *P. falciparum* sporozoites were obtained from NF54 strain as described in Ponnodurai *et al*. (1989) and from mosquitoes fed with gametocytes produced *in vitro* from Thai patient isolates (Galey et al., 1990). *P. falciparum* liver schizonts were identified in liver biopsies of a Sapajou monkey (*Cebus apella*, in day 5 post-sporozoite challenge) infected with the African isolate 730XI (Druilhe *et al*., 1984), and of a chimpanzee (*Pan troglodytes,* in day 6 post-sporozoite challenge) infected with NF54 strain (Meis *et al*., 1990).

### 2. Nucleic acid isolation and hybridisation

Parasite genomic DNA was purified from saponin-lysed infected erythrocytes (Robson *et al*., 1991). Total RNA from sporozoites and parasite blood stages were extracted according to Chomczynski *et al*. (1987). DNA probes were randomly [³²P]-radiolabelled according to the manufacturer's recommendations (Amersham, UK). Southern and Northern blottings, probe hybridisations and washes were performed on 5-10 µg of material by standard methods (Sambrook *et al*., 1989).
Low stringency cross-species hybridisations were performed overnight at 54°C in: 5x Denhardt's solution, 6x SSC buffer, 0.1 % SDS, 0.1 mg/ml sonicated salmon sperm DNA. Membranes were washed 30 min. at 54°C in 0.2X or 0.1X SSC buffer before autoradiography.

### 3. Cloning and sequencing protocols

A size-selected (0.5-1.5 Kb) genomic expression library was prepared in the phage λgt11 from *P. falciparum* T9/96 DNA and differentially screened with various stage-restricted sera as previously described (Guérin-Marchand *et al*., 1987). λgt11-DG729 and -DG679 DNA were prepared from a liquid phage lysate. The gel-purified *Eco*RI inserts were cloned into plasmid pUC18 and sequenced. The DG729 insert was randomly radiolabelled and used as a probe to screen an *Eco*RI-digested genomic DNA library prepared from the K1 strain in the phage λgt10 (generously provided by G. Langsley, Pasteur Institute). Five positive clones were isolated and analysed. One of them, clone k1.2, was found to contain the largest *Eco*RI insert and was therefore chosen for subcloning and complete sequence analysis. This 6.7 Kb *Eco*RI fragment and subclones derived from it (spanning the entire insert) were cloned into pUC18. A series of Exonuclease III-digested subclones from the 1.8 Kb repeated regions R1-R2 of clone k1.2 was obtained using the Erase-a-Base Kit (Promega, U.S.A.). All clones and subclones described above were sequenced on both strands with insert flanking or internal oligonucleotidic primers using the dideoxy method (Sanger *et al*., 1977) and the Sequenase enzyme system (United States Biochemicals Corp.).

### 4. PCR and RT-PCR amplifications

RT-PCR experiments were performed on 300-500 ng of total RNA (for blood stage parasites) or on the RNA pellet obtained from 10⁶-10⁷ NF54 sporozoites. cDNA were synthesized from 30 pmoles of primers S2(-) by the MMLV-reverse transcriptase in a final volume of 20 µl according to the manufacturer 's recommendations (Gibco-BRL). PCR reactions were carried out on 10 µl of cDNA synthesis reaction or on 1 µg of genomic DNA, according to the manufacturer 's recommendations (Amersham, UK).
For *lsa-3* amplification in human blood samples and *P. falciparum* detection in challenged chimpanzees, PCR was performed as described in Bottius *et al*. (1996) where primers described within for clone DG157 correspond to primers S1 and S2 reported here.

### 5. Peptides synthesis and production of recombinant proteins

Peptides and lipopeptides used for chimpanzee immunisations were synthesized as described in Ben Mohamed *et al*. (1997). All peptides and lipopeptides were purified over 90% by reversed-phase chromatography, the impurities essentially consisting in shorter sequences. Long synthetic peptides GP5 (aa 1241-1346), GP6 (aa 1143-1255), GP8 (aa 1026-1095) and GP11 (aa 840-907) were synthesized as described in Roggero *et al*. (1995); they are all located in region NR-B (strain K1), i.e. the non-repeated region of PC insert.
Recombinant protein β-729 was prepared from a liquide lysate as described in Guérin-Marchand *et al*. (1987). Control GST protein and GST-fused recombinant proteins were prepared according to the manufacturer 's recommendations (Invitrogen) except for GST-PC which was prepared from 20 liter cultures due to poor production yields. This large scale culture was incubated until OD₆₀₀= 8.0; bacteria were then pelleted, lysed using a French Press and filtered before standard purification.

### 6. Antibodies and antisera

Human antibodies were immunopurified on recombinant proteins and peptides as previously described in Marchand & Druilhe (1990) and Brahimi *et al*. (1993), respectively. Mouse and chimpanzee anti-NR2 peptide antibodies were induced respectively in mice and in chimpanzee Gerda by lipopeptide NR2 injections as described in Ben Mohamed *et al*. (1997). Mouse antisera against GST-PC recombinant protein and long peptides GP5-6-8-11 (used for Western blotting) were obtained following 3 subcutaneous injections of the immunogen (100 µg) emulsified in SBAS2 adjuvant (Stoute *et al*., 1997).

### 7. Western blot analysis

Proteins from intraerythrocytic parasites and sporozoites were solubilized in sodium dodecyl sulphate (SDS)-containing sample buffer, subjected to 5% SDS-polyacrylamide gel electrophoresis under reducing conditions, electroblotted onto nitrocellulose membrane and detected as described previously (Bouharoun-Tayoun & Druilhe, 1992), using mouse antibodies (at dilution 1/100). Visualisation was performed by peroxidase-conjugated goat anti-human IgG and chemoluminescence (ECL Western blotting reagents, Amersham).

### 8. Immunofluorescence Antibody Test (IFAT)

IFAT were performed as described previously (Druilhe *et al*., 1986) on asynchronous erythrocytic cultures of *P. falciparum* NF54 strain, on freshly dissected live sporozoites labelled in suspension, on wet sporozoites deposited on poly-L-lysine-coated slides and on glutaraldehyde-fixed sporozoites, as well as on Carnoy-fixed liver schizonts. Positive IFAT on liver schizonts were verified by phase contrast microscopy and subsequent Giemsa staining of the sections (Druilhe *et al*., 1984).
Ahlborg, N., *et al*. Definition of the epitope recognized by the *Plasmodium falciparum-*reactive human monoclonal antibody 33G2. *Mol. Biochem. Parasitol*., **46,** 89-95 (1991).
Aidoo, M., *et al*. CTL epitopes for HLA-B53 and other HLA types in the malaria vaccine candidate Liver Stage Antigen-3. *Infect. Immun*., **68,** 227-232 (2000).
Barnes, D. A., *et al. Plasmodium falciparum*. D260, an intraerythrocytic parasite protein, is a member of the glutamic acid dipeptide-repeat family of proteins. *Experim. Parasitol.*, **81,** 79-89 (1995).
Ben Mohamed, L., *et al*. Lipopeptide immunization without adjuvant induces potent and long-lasting B, T helper, and cytotoxic T lymphocyte responses against a malaria liver stage antigen in mice and chimpanzees. *Eur. J. Immunol*., **27,** 1242-1253 (1997).
Bottius, E., *et al*. Malaria - even more chronic in nature than previously thought - evidence for subpatent parasitaemia detectable by the polymerase chain reaction. *Trans. Roy. Soc. Trop. Med. Hyg.*, **90,** 15-19 (1996).
Bouharoun-Tayoun, H. & Druilhe, P. *Plasmodium falciparum* malaria: evidence for an isotype imbalance which may be responsible for delayed acquisition of protective immunity. *Infect. Immun*., **60,** 1473-1481 (1992).
Brahimi, K., *et al*. Fast immunopurification of small amounts of specific antibodies on peptides bound to ELISA plates. J. *Immunol. Methods,* **162,** 69-75 (1993).
Chomczynski, P. & Sacchi, N. Single step method of RNA isolation by acid guanidinium thiocyanate-phenol-chloroform extraction. *Anal. Biochem*., **162,** 156-161 (1987).
Druilhe, P., *et al*. Species- and stage-specific antigens in exoerythrocytic stages of *Plasmodium falciparum. Am. J. Trop. Med*. *Hyg*., **33,** 336-341 (1984).
Druilhe, P., *et al*. Levels of antibodies to *Plasmodium falciparum* sporozoite surface antigens reflect malaria transmission rates and are persistent in the absence of reinfections. *Infect. Immun*., **53,** 393-397 (1986).
Druilhe, P., *et al*. A primary malaria infection is composed of a very wide range of genetically diverse but related parasites. *J. Clin. Invest*., **101,** 1-9 (1998).
Gaboriaud, C., *et al.* Hydrophobic cluster analysis: an efficient new way to compare and analyse amino acid sequences. *FEBS Lett.*, **224,** 149-155 (1987).
Galey, B., *et al*. Evidence for diversity of *Plasmodium falciparum* sporozoite surface antigens derived from analysis of antibodies elicited in humans. *Infect. Immun*., **58,** 2995-3001 (1990).
Gardner, M. J., *et al*. Chromosome 2 sequence of the human malaria parasite *Plasmodium falciparum*. *Science,* **282,** 1126-1132 (1998).
Guérin-Marchand, C., *et al*. A liver stage-specific antigen of *Plasmodium falciparum* characterized by gene cloning. *Nature (London),* **329,** 164-167 (1987).
Hernandez-Rivas, R., *et al*. Compartmentalization of genes coding for immunodominant antigens to fragile chromosome ends leads to dispersed subtelomeric gene families and rapid gene evolution in *Plasmodium falciparum*. *Mol. Biochem. Parasitol*., **78,** 137-48 (1996).
Meis, J. F. G. M., *et al*. *Plasmodium falciparum*. studies on mature exoerythrocytic forms in the liver of the chimpanzee, *Pan troglodytes*. *Exp. Parasitol*., **70,** 1-11 (1990).
Moelans, I. I. M. D. & Schoenmakers, J. G. G. Crossreactive antigens between life cycle stages of *Plasmodium falciparum*. *Parasitol*. *Today*, **8,** 118-123 (1992).
Nielsen, H., *et al*. Identification of prokaryotic and eukaryotic signal peptides and prediction of their clivage sites. *Prot*. *Engineering*, **10**, 1-6 (1997).
Ponnudurai, T., *et al*. Chloroquine sensitivity of isolates of *Plasmodium falciparum* adapted to *in vitro* culture. *Trop. Geo. Med.*, **33,** 50-4 (1981).
Ponnudurai, T., *et al.* Sporozoite load of mosquitoes infected with *Plasmodium falciparum. Trans. Roy. Soc. Trop. Med. Hyg*., **83,** 67-70 (1989).
Robson, K. J. H. & Jennings, M. W. The structure of the calmodulin gene of *Plasmodium falciparum. Mol. Biochem. Parasitol.*, **46**, 19-34 (1991).
Roggero, M.A., *et al*. Synthesis and immunological characterization of 104-mer and 102-mer peptides corresponding to the N-and C-terminal regions of the *Plasmodium falciparum* CS Protein. *Mol. Immunol*., **32,** 1301-1309 (1995).
Sambrook, J., *et al*. Molecular cloning. A laboratory manual-2nd edition. Cold Spring Harbor, New York, Cold Spring Harbor Laboratory Press (1989).
Sanger, F., *et al*. DNA sequencing with chain-terminating inhibitors. *Proc. Natl. Acad. Sci. U.S.A.*, **74,** 5463-5467 (1977).
Stoute, J.A., *et al*. A preliminary evaluation of a recombinant Circumsporozoite Protein vaccine against *Plasmodium falciparum* malaria. *New Engl. J. Med*., **336,** 86-91 (1997).
Thaithong, S. & Beale, G. H. Resistance of ten Thai isolates of *Plasmodium falciparum* to chloroquine and pyrimethamine by in vitro tests. *Trans. Roy. Soc. Trop. Med. Hyg*., **75,** 271-3 (1981).
Thaithong, S., *et al*. Clonal diversity in a single isolate of the malaria parasite *Plasmodium falciparum. Trans. Roy. Soc. Trop*. *Med. Hyg*., **78,** 242-5 (1984).
Trager, W. & Jensen, J. B. Human malaria parasites in continuous culture. *Science*, **193,** 673-675 (1976).
Wilkins, M.R. *et al*. Detailed peptide characterisation using PEPTIDEMASS - a World Wide Web accessible tool. *Electrophoresis*, **18,** 403-408 (1997).

## Claims

1. A vaccine composition comprising a Th1-inducing adjuvant in combination with a protecting Liver Stage Antigen or immunological fragment thereof of a human malaria parasite with the proviso that when the immunological fragment is an immunological fragment of LSA-3, the Th1-inducing adjuvant is not Montanide.

2. A vaccine composition as claimed in claim 1 wherein the human malaria parasite is Plasmodium falciparum.

3. A vaccine composition as claimed in claim 1 or claim 2 in which the Thl-inducing adjuvant comprises QS21, De-O-acylated monophosphoryl lipid A (3D-MPL) and an oil in water emulsion wherein the oil in water emulsion has the following composition: a metabolisable oil, such a squalene, alpha tocopherol and tween 80.

4. A vaccine composition as claimed in claim 1 or 2 or claim 3 wherein said protecting Liver Stage Antigen is the Liver Stage Antigen 3 (LSA-3) or immunological fragment thereof.

5. A vaccine composition according to any one of claims 1 to 4 comprising in addition at least one other protecting antigen or an immunological fragment thereof, of a malaria parasite.

6. A vaccine composition as claimed in claim 4 in which the other malaria antigen is selected from the following group:
a) a hybrid protein comprising substantially all the C-terminal portion of the CS protein, four or more tandem repeats of the immunodominant region, and the surface antigen from hepatitis B virus (HBsAg), in particular RTS,S, or immunogenic derivatives including fragments thereof;
b) the TRAP protein of the T9/96 isolate of Plasmodium falciparum and proteins having at least 80% homology thereto and immunogenic derivatives including fragments thereof;
c) the MSP-1 of Plasmodium falciparum or Plasmodium vivax and proteins having at least 80% homology thereto and immunogenic derivatives including fragments thereof; and
d) the MSP-3 of Plasmodium falciparum or Plasmodium vivax and proteins having at least 70% homology with the C-terminal region thereof, and immunogenic derivatives including fragments thereof.

7. A vaccine composition according to claims 1 to 6 capable of involving a T cell response in a mammal to the antigen or antigenic composition

8. A vaccine composition according to claims 1 to 7 capable of stimulating interferon γ production.

9. A vaccine composition according to claims 1 to 8, wherein the ratio of QS21:3D-MPL is from 1:10 to 10:1.

10. A vaccine composition according to claims 1 to 8, wherein the ratio of QS21:3D-MPL is from 1:1 to 1:2.5.

11. A process to make a vaccine composition according to any one of claims 1 to 10 comprising admixing QS21, 3D-MPL and the oil in water emulsion as defined in claim 2 with a protecting Liver Stage Antigen of a human malaria parasite.

12. A process according to claim 11 wherein the Liver Stage Antigen is LSA-3 of Plasmodium falciparum or immunological fragment thereof.

13. Use of a composition according to any one of claims 1 to 10 for the prophylaxis or treatment of malaria infections.


### Annex to the application documents - subsequently filed sequences listing

SEQUENCE LISTING

<110> SmithKline Beecham Biologicals S.A.

<120> Immunogenic compositions comprising
  Liver Stage Malarial Antigens

<130> B45250

<160> 8

<170> FastSEQ for Windows Version 3.0

<210> 1
<211> 5529
<212> DNA
<213> K1 Parasite Strain

<400> 1
atgacaaata gtaattacaa atcaaataat aaaacatata atgaaaataa taatgaacaa 60
ataactacca tatttaatag aacaaatatg aatccgataa aaaaatgtca tatgagagaa 120
aaaataaata agtacttttt tttgatcaaa attttgacat gcaccatttt aatatgggct 180
gtacaatatg ataataacgt aagataaaaa actaaataat aaatataaat aaaaaaaaaa 240
aaaaaaaaaa aaaaatcaac tatatagtat gtataatata tatatatata tatatatata 300
tatatatata tatatattta tttttattta tttattaatt tttttttttt tatattatct 360
ttttagtctg atataaacaa gagttggaaa aaaaatacgt atgtagataa gaaattgaat 420
aaactattta acagaagttt aggagaatct caagtaaatg gtgaattagc tagtgaagaa 480
gtaaaggaaa aaattcttga cttattagaa gaaggaaata cattaactga aagtgtagat 540
gataataaaa atttagaaga agccgaagat ataaaggaaa atatcttatt aagtaatata 600
gaagaaccaa aagaaaatat tattgacaat ttattaaata atattggaca aaattcagaa 660
aaacaagaaa gtgtatcaga aaatgtacaa gtcagtgatg aactttttaa tgaattatta 720
aatagtgtag atgttaatgg agaagtaaaa gaaaatattt tggaggaaag tcaagttaat 780
gacgatattt ttaatagttt agtaaaaagt gttcaacaag aacaacaaca caatgttgaa 840
gaaaaagttg aagaaagtgt agaagaaaat gacgaagaaa gtgtagaaga aaatgtagaa 900
gaaaatgtag aagaaaatga cgacggaagt gtagcctcaa gtgttgaaga aagtatagct 960
tcaagtgttg atgaaagtat agattcaagt attgaagaaa atgtagctcc aactgttgaa 1020
gaaatcgtag ctccaagtgt tgtagaaagt gtggctccaa gtgttgaaga aagtgtagaa 1080
gaaaatgttg aagaaagtgt agctgaaaat gttgaagaaa gtgtagctga aaatgttgaa 1140
gaaagtgtag ctgaaaatgt tgaagaaagt gtagctgaaa atgttgaaga aatcgtagct 1200
ccaactgttg aagaaatcgt agctccaact gttgaagaaa ttgtagctcc aagtgttgta 1260
gaaagtgtgg ctccaagtgt tgaagaaagt gtagaagaaa atgttgaaga aagtgtagct 1320
gaaaatgttg aagaaagtgt agctgaaaat gttgaagaaa gtgtagctga aaatgttgaa 1380
gaaagtgtag ctgaaaatgt tgaagaaagt gtagctgaaa atgttgaaga aatcgtagct 1440
ccaactgttg aagaaatcgt agctccaact gttgaagaaa ttgtagctcc aagtgttgta 1500
gaaagtgtgg ctccaagtgt tgaagaaagt gtagaagaaa atgttgaaga aagtgtagct 1560
gaaaatgttg aagaaagtgt agctgaaaat gttgaagaaa gtgtagctga aaatgttgaa 1620
gaaagtgtag ctgaaaatgt tgaagaaagt gtagctgaaa atgttgaaga aagtgtagct 1680
gaaaatgttg aagaaagtgt agctgaaaat gttgaagaaa tcgtagctcc aactgttgaa 1740
gaaatcgtag ctccaactgt tgaagaaatt gtagctccaa gtgttgtaga aagtgtggct 1800
ccaagtgttg aagaaagtgt agaagaaaat gttgaagaaa gtgtagctga aaatgttgaa 1860
gaaagtgtag ctgaaaatgt tgaagaaagt gtagctgaaa atgttgaaga aagtgtagct 1920
gaaaatgttg aagaaatcgt agctccaact gttgaagaaa tcgtagctcc aactgttgaa 1980
gaaattgtag ctccaagtgt tgtagaaagt gtggctccaa gtgttgaaga aagtgtagaa 2040
gaaaatgttg aagaaagtgt agctgaaaat gttgaagaaa gtgtagctga aaatgttgaa 2100
gaaagtgtag ctgaaaatgt tgaagaaatc gtagctccaa ctgttgaaga aatcgtagct 2160
ccaactgttg aagaaattgt agctccaagt gttgtagaaa gtgtggctcc aagtgttgaa 2220
gaaagtgtag aagaaaatgt tgaagaaagt gtagctgaaa atgttgaaga aagtgtagct 2280
gaaaatgttg aagaaagtgt agctgaaaat gttgaagaaa gtgtagctga aaatgttgaa 2340
gaaatcgtag ctccaactgt tgaagaaatc gtagctccaa ctgttgaaga aattgtagct 2400
ccaagtgttg tagaaagtgt ggctccaagt gttgaagaaa gtgtagaaga aaatgttgaa 2460
gaaagtgtag ctgaaaatgt tgaagaaagt gtagctgaaa atgttgaaga aagtgtagct 2520
gaaaatgttg aagaaagtgt agctccaact gttgaagaaa ttgtagctcc aagtgttgaa 2580

gaaagtgtag ctccaagtgt tgaagaaagt gttgctgaaa acgttgcaac aaatttatca 2640
gacaatcttt taagtaattt attaggtggt atcgaaactg aggaaataaa ggacagtata 2700
ttaaatgaga tagaagaagt aaaagaaaat gtagtcacca caatactaga aaacgtagaa 2760
gaaactacag ctgaaagtgt aactactttt agtaacatat tagaggagat acaagaaaat 2820
actattacta atgatactat agaggaaaaa ttagaagaac tccacgaaaa tgtattaagt 2880
gccgctttag aaaataccca aagtgaagag gaaaagaaag aagtaataga tgtaattgaa 2940
gaagtaaaag aagaggtcgc taccacttta atagaaactg tggaacaggc agaagaaaag 3000
agcgcaaata caattacgga aatatttgaa aatttagaag aaaatgcagt agaaagtaat 3060
gaaaatgttg cagagaattt agagaaatta aacgaaactg tatttaatac tgtattagat 3120
aaagtagagg aaacagtaga aattagcgga gaaagtttag aaaacaatga aatggataaa 3180
gcatttttta gtgaaatatt tgataatgta aaaggaatac aagaaaattt attaacaggt 3240
atgtttcgaa gtatagaaac cagtatagta atccaatcag aagaaaaggt tgatttgaat 3300
gaaaatgtgg ttagttcgat tttagataat atagaaaata tgaaagaagg tttattaaat 3360
aaattagaaa atatttcaag tactgaaggt gttcaagaaa ctgtaactga acatgtagaa 3420
caaaatgtat atgtggatgt tgatgttcct gctatgaaag atcaattttt aggaatatta 3480
aatgaggcag gagggttgaa agaaatgttt tttaatttgg aagatgtatt taaaagtgaa 3540
agtgatgtaa ttactgtaga agaaattaag gatgaaccgg ttcaaaaaga ggtagaaaaa 3600
gaaactgtta gtattattga agaaatggaa gaaaatattg tagatgtatt agaggaagaa 3660
aaagaagatt taacagacaa gatgatagat gcagtagaag aatccataga aatatcttca 3720
gattctaaag aagaaactga atctattaaa gataaagaaa aagatgtttc actagttgtt 3780
gaagaagttc aagacaatga tatggatgaa agtgttgaga aagttttaga attgaaaaat 3840
atggaagagg agttaatgaa ggatgctgtt gaaataaatg acattactag caaacttatt 3900
gaagaaactc aagagttaaa tgaagtagaa gcagatttaa taaaagatat ggaaaaatta 3960
aaagaattag aaaaagcatt atcagaagat tctaaagaaa taatagatgc aaaagatgat 4020
acattagaaa aagttattga agaggaacat gatataacga cgacgttgga tgaagttgta 4080
gaattaaaag atgtcgaaga agacaagatc gaaaaagtat ctgatttaaa agatcttgaa 4140
gaagatatat taaaagaagt aaaagaaatc aaagaacttg aaagtgaaat tttagaagat 4200
tataaagaat taaaaactat tgaaacagat attttagaag agaaaaaaga aatagaaaaa 4260
gatcattttg aaaaattcga agaagaagct gaagaaataa aagatcttga agcagatata 4320
ttaaaagaag tatcttcatt agaagttgaa gaagaaaaaa aattagaaga agtacacgaa 4380
ttaaaagaag aggtagaaca tataataagt ggtgatgcgc atataaaagg tttggaagaa 4440
gatgatttag aagaagtaga tgatttaaaa ggaagtatat tagacatgtt aaaggaagat 4500
atggaattag gggatatgga taaggaaagt ttagaagatg taacaacaaa acttggagaa 4560
agagttgaat ccttaaaaga tgttttatct agtgcattag gcatggatga agaacaaatg 4620
aaaacaagaa aaaaagctca aagacctaag ttggaagaag tattattaaa agaagaggtt 4680
aaagaagaac caaagaaaaa aataacaaaa aagaaagtaa ggtttgatat taaggataag 4740
gaaccaaaag atgaaatagt agaagttgaa atgaaagatg aagatataga agaagatgta 4800
gaagaagata tagaagaaga tatagaagaa gataaagttg aagatataga tgaagatata 4860
gatgaagata taggtgaaga caaagatgaa gttatagatt taatagtcca aaaagagaaa 4920
cgcattgaaa aggttaaagc gaaaaagaaa aaattagaaa aaaaagttga agaaggtgtt 4980
agtggtctta aaaaacacgt agacgaagta atgaaatatg ttcaaaaaat tgataaagaa 5040
gttgataaag aagtatctaa agctttagaa tcaaaaaatg atgttactaa tgttttaaaa 5100
caaaatcaag atttttttag taaagttaaa aacttcgtaa aaaaatataa agtatttgct 5160
gcaccattca tatctgccgt tgcagcattt gcatcatatg tagttgggtt ctttacattt 5220
tctttatttt catcatgtgt aacaatagct tcttcaactt acttattatc aaaagttgac 5280
aaaactataa ataaaaataa ggagagaccg ttttattcat ttgtatttga tatctttaag 5340
aatttaaaac attatttaca acaaatgaaa gaaaaattta gtaaagaaaa aaataataat 5400
gtaatagaag taacaaacaa agctgagaaa aaaggtaatg tacaggtaac aaataaaacc 5460
gagaaaacaa ctaaagttga taaaaataat aaagtaccga aaaaaagaag aacgcaaaaa 5520
tcaaaataa 5529

<210> 2
<211> 1787
<212> PRT
<213> K1 parasite clone

<400> 2
Met Thr Asn Ser Asn Tyr Lys Ser Asn Asn Lys Thr Tyr Asn Glu Asn
1               5                   10                  15
Asn Asn Glu Gln Ile Thr Thr Ile Phe Asn Arg Thr Asn Met Asn Pro
            20                  25                  30
Ile Lys Lys Cys His Met Arg Glu Lys Ile Asn Lys Tyr Phe Phe Leu

35                  40                  45
Ile Lys Ile Leu Thr Cys Thr Ile Leu Ile Trp Ala Val Gln Tyr Asp
    50                  55                  60
Asn Asn Ser Asp Ile Asn Lys Ser Trp Lys Lys Asn Thr Tyr Val Asp
65                  70                  75                  80
Lys Lys Leu Asn Lys Leu Phe Asn Arg Ser Leu Gly Glu Ser Gln Val
                85                  90                  95
Asn Gly Glu Leu Ala Ser Glu Glu Val Lys Glu Lys Ile Leu Asp Leu
            100                 105                 110
Leu Glu Glu Gly Asn Thr Leu Thr Glu Ser Val Asp Asp Asn Lys Asn
        115                 120                 125
Leu Glu Glu Ala Glu Asp Ile Lys Glu Asn Ile Leu Leu Ser Asn Ile
    130                 135                 140
Glu Glu Pro Lys Glu Asn Ile Ile Asp Asn Leu Leu Asn Asn Ile Gly
145                 150                 155                 160
Gln Asn Ser Glu Lys Gln Glu Ser Val Ser Glu Asn Val Gln Val Ser
                165                 170                 175
Asp Glu Leu Phe Asn Glu Leu Leu Asn Ser Val Asp Val Asn Gly Glu
            180                 185                 190
Val Lys Glu Asn Ile Leu Glu Glu Ser Gln Val Asn Asp Asp Ile Phe
        195                 200                 205
Asn Ser Leu Val Lys Ser Val Gln Gln Glu Gln Gln His Asn Val Glu
    210                 215                 220
Glu Lys Val Glu Glu Ser Val Glu Glu Asn Asp Glu Glu Ser Val Glu
225                 230                 235                 240
Glu Asn Val Glu Glu Asn Val Glu Glu Asn Asp Asp Gly Ser Val Ala
                245                 250                 255
Ser Ser Val Glu Glu Ser Ile Ala Ser Ser Val Asp Glu Ser Ile Asp
            260                 265                 270
Ser Ser Ile Glu Glu Asn Val Ala Pro Thr Val Glu Glu Ile Val Ala
        275                 280                 285
Pro Ser Val Val Glu Ser Val Ala Pro Ser Val Glu Glu Ser Val Glu
    290                 295                 300
Glu Asn Val Glu Glu Ser Val Ala Glu Asn Val Glu Glu Ser Val Ala
305                 310                 315                 320
Glu Asn Val Glu Glu Ser Val Ala Glu Asn Val Glu Glu Ser Val Ala
                325                 330                 335
Glu Asn Val Glu Glu Ile Val Ala Pro Thr Val Glu Glu Ile Val Ala
            340                 345                 350
Pro Thr Val Glu Glu Ile Val Ala Pro Ser Val Val Glu Ser Val Ala
        355                 360                 365
Pro Ser Val Glu Glu Ser Val Glu Glu Asn Val Glu Glu Ser Val Ala
    370                 375                 380
Glu Asn Val Glu Glu Ser Val Ala Glu Asn Val Glu Glu Ser Val Ala
385                 390                 395                 400
Glu Asn Val Glu Glu Ser Val Ala Glu Asn Val Glu Glu Ser Val Ala
                405                 410                 415
Glu Asn Val Glu Glu Ile Val Ala Pro Thr Val Glu Glu Ile Val Ala
            420                 425                 430
Pro Thr Val Glu Glu Ile Val Ala Pro Ser Val Val Glu Ser Val Ala
        435                 440                 445
Pro Ser Val Glu Glu Ser Val Glu Glu Asn Val Glu Glu Ser Val Ala
    450                 455                 460
Glu Asn Val Glu Glu Ser Val Ala Glu Asn Val Glu Glu Ser Val Ala
465                 470                 475                 480
Glu Asn Val Glu Glu Ser Val Ala Glu Asn Val Glu Glu Ser Val Ala
                485                 490                 495
Glu Asn Val Glu Glu Ser Val Ala Glu Asn Val Glu Glu Ser Val Ala
            500                 505                 510
Glu Asn Val Glu Glu Ile Val Ala Pro Thr Val Glu Glu Ile Val Ala
        515                 520                 525
Pro Thr Val Glu Glu Ile Val Ala Pro Ser Val Val Glu Ser Val Ala
    530                 535                 540
Pro Ser Val Glu Glu Ser Val Glu Glu Asn Val Glu Glu Ser Val Ala

545                 550                 555                 560
Glu Asn Val Glu Glu Ser Val Ala Glu Asn Val Glu Glu Ser Val Ala
                565                 570                 575
Glu Asn Val Glu Glu Ser Val Ala Glu Asn Val Glu Glu Ile Val Ala
            580                 585                 590
Pro Thr Val Glu Glu Ile Val Ala Pro Thr Val Glu Glu Ile Val Ala
        595                 600                 605
Pro Ser Val Val Glu Ser Val Ala Pro Ser Val Glu Glu Ser Val Glu
    610                 615                 620
Glu Asn Val Glu Glu Ser Val Ala Glu Asn Val Glu Glu Ser Val Ala
625                 630                 635                 640
Glu Asn Val Glu Glu Ser Val Ala Glu Asn Val Glu Glu Ile Val Ala
                645                 650                 655
Pro Thr Val Glu Glu Ile Val Ala Pro Thr Val Glu Glu Ile Val Ala
            660                 665                 670
Pro Ser Val Val Glu Ser Val Ala Pro Ser Val Glu Glu Ser Val Glu
        675                 680                 685
Glu Asn Val Glu Glu Ser Val Ala Glu Asn Val Glu Glu Ser Val Ala
    690                 695                 700
Glu Asn Val Glu Glu Ser Val Ala Glu Asn Val Glu Glu Ser Val Ala
705                 710                 715                 720
Glu Asn Val Glu Glu Ile Val Ala Pro Thr Val Glu Glu Ile Val Ala
                725                 730                 735
Pro Thr Val Glu Glu Ile Val Ala Pro Ser Val Val Glu Ser Val Ala
            740                 745                 750
Pro Ser Val Glu Glu Ser Val Glu Glu Asn Val Glu Glu Ser Val Ala
        755                 760                 765
Glu Asn Val Glu Glu Ser Val Ala Glu Asn Val Glu Glu Ser Val Ala
    770                 775                 780
Glu Asn Val Glu Glu Ser Val Ala Pro Thr Val Glu Glu Ile Val Ala
785                 790                 795                 800
Pro Ser Val Glu Glu Ser Val Ala Pro Ser Val Glu Glu Ser Val Ala
                805                 810                 815
Glu Asn Val Ala Thr Asn Leu Ser Asp Asn Leu Leu Ser Asn Leu Leu
            820                 825                 830
Gly Gly Ile Glu Thr Glu Glu Ile Lys Asp Ser Ile Leu Asn Glu Ile
        835                 840                 845
Glu Glu Val Lys Glu Asn Val Val Thr Thr Ile Leu Glu Asn Val Glu
    850                 855                 860
Glu Thr Thr Ala Glu Ser Val Thr Thr Phe Ser Asn Ile Leu Glu Glu
865                 870                 875                 880
Ile Gln Glu Asn Thr Ile Thr Asn Asp Thr Ile Glu Glu Lys Leu Glu
                885                 890                 895
Glu Leu His Glu Asn Val Leu Ser Ala Ala Leu Glu Asn Thr Gln Ser
            900                 905                 910
Glu Glu Glu Lys Lys Glu Val Ile Asp Val Ile Glu Glu Val Lys Glu
        915                 920                 925
Glu Val Ala Thr Thr Leu Ile Glu Thr Val Glu Gln Ala Glu Glu Lys
    930                 935                 940
Ser Ala Asn Thr Ile Thr Glu Ile Phe Glu Asn Leu Glu Glu Asn Ala
945                 950                 955                 960
Val Glu Ser Asn Glu Asn Val Ala Glu Asn Leu Glu Lys Leu Asn Glu
                965                 970                 975
Thr Val Phe Asn Thr Val Leu Asp Lys Val Glu Glu Thr Val Glu Ile
            980                 985                 990
Ser Gly Glu Ser Leu Glu Asn Asn Glu Met Asp Lys Ala Phe Phe Ser
        995                 1000                1005
Glu Ile Phe Asp Asn Val Lys Gly Ile Gln Glu Asn Leu Leu Thr Gly
    1010                1015                1020
Met Phe Arg Ser Ile Glu Thr Ser Ile Val Ile Gln Ser Glu Glu Lys
1025                1030                1035                104
Val Asp Leu Asn Glu Asn Val Val Ser Ser Ile Leu Asp Asn Ile Glu
                1045                1050                1055
Asn Met Lys Glu Gly Leu Leu Asn Lys Leu Glu Asn Ile Ser Ser Thr

1060                 1065                 1070
Glu Gly Val Gln Glu Thr Val Thr Glu His Val Glu Gln Asn Val Tyr
        1075                 1080                 1085
Val Asp Val Asp Val Pro Ala Met Lys Asp Gln Phe Leu Gly Ile Leu
    1090                 1095                 1100
Asn Glu Ala Gly Gly Leu Lys Glu Met Phe Phe Asn Leu Glu Asp Val
1105                 1110                 1115                 112
Phe Lys Ser Glu Ser Asp Val Ile Thr Val Glu Glu Ile Lys Asp Glu
                1125                 1130                 1135
Pro Val Gln Lys Glu Val Glu Lys Glu Thr Val Ser Ile Ile Glu Glu
            1140                 1145                 1150
Met Glu Glu Asn Ile Val Asp Val Leu Glu Glu Glu Lys Glu Asp Leu
        1155                 1160                 1165
Thr Asp Lys Met Ile Asp Ala Val Glu Glu Ser Ile Glu Ile Ser Ser
    1170                 1175                 1180
Asp Ser Lys Glu Glu Thr Glu Ser Ile Lys Asp Lys Glu Lys Asp Val
1185                 1190                 1195                 120
Ser Leu Val Val Glu Glu Val Gln Asp Asn Asp Met Asp Glu Ser Val
                1205                 1210                 1215
Glu Lys Val Leu Glu Leu Lys Asn Met Glu Glu Glu Leu Met Lys Asp
            1220                 1225                 1230
Ala Val Glu Ile Asn Asp Ile Thr Ser Lys Leu Ile Glu Glu Thr Gln
        1235                 1240                 1245
Glu Leu Asn Glu Val Glu Ala Asp Leu Ile Lys Asp Met Glu Lys Leu
    1250                 1255                 1260
Lys Glu Leu Glu Lys Ala Leu Ser Glu Asp Ser Lys Glu Ile Ile Asp
1265                 1270                 1275                 128
Ala Lys Asp Asp Thr Leu Glu Lys Val Ile Glu Glu Glu His Asp Ile
                1285                 1290                 1295
Thr Thr Thr Leu Asp Glu Val Val Glu Leu Lys Asp Val Glu Glu Asp
            1300                 1305                 1310
Lys Ile Glu Lys Val Ser Asp Leu Lys Asp Leu Glu Glu Asp Ile Leu
        1315                 1320                 1325
Lys Glu Val Lys Glu Ile Lys Glu Leu Glu Ser Glu Ile Leu Glu Asp
    1330                 1335                 1340
Tyr Lys Glu Leu Lys Thr Ile Glu Thr Asp Ile Leu Glu Glu Lys Lys
1345                 1350                 1355                 136
Glu Ile Glu Lys Asp His Phe Glu Lys Phe Glu Glu Glu Ala Glu Glu
                1365                 1370                 1375
Ile Lys Asp Leu Glu Ala Asp Ile Leu Lys Glu Val Ser Ser Leu Glu
            1380                 1385                 1390
Val Glu Glu Glu Lys Lys Leu Glu Glu Val His Glu Leu Lys Glu Glu
        1395                 1400                 1405
Val Glu His Ile Ile Ser Gly Asp Ala His Ile Lys Gly Leu Glu Glu
    1410                 1415                 1420
Asp Asp Leu Glu Glu Val Asp Asp Leu Lys Gly Ser Ile Leu Asp Met
1425                 1430                 1435                 144
Leu Lys Gly Asp Met Glu Leu Gly Asp Met Asp Lys Glu Ser Leu Glu
                1445                 1450                 1455
Asp Val Thr Thr Lys Leu Gly Glu Arg Val Glu Ser Leu Lys Asp Val
            1460                 1465                 1470
Leu Ser Ser Ala Leu Gly Met Asp Glu Glu Gln Met Lys Thr Arg Lys
        1475                 1480                 1485
Lys Ala Gln Arg Pro Lys Leu Glu Glu Val Leu Leu Lys Glu Glu Val
    1490                 1495                 1500
Lys Glu Glu Pro Lys Lys Lys Ile Thr Lys Lys Lys Val Arg Phe Asp
1505                 1510                 1515                 152
Ile Lys Asp Lys Glu Pro Lys Asp Glu Ile Val Glu Val Glu Met Lys
                1525                 1530                 1535
Asp Glu Asp Ile Glu Glu Asp Val Glu Glu Asp Ile Glu Glu Asp Ile
            1540                 1545                 1550
Glu Glu Asp Lys Val Glu Asp Ile Asp Glu Asp Ile Asp Glu Asp Ile
        1555                 1560                 1565
Gly Glu Asp Lys Asp Glu Val Ile Asp Leu Ile Val Gln Lys Glu Lys

1570                 1575                 1580
Arg Ile Glu Lys Val Lys Ala Lys Lys Lys Lys Leu Glu Lys Lys Val
1585                 1590                 1595                 160
Glu Glu Gly Val Ser Gly Leu Lys Lys His Val Asp Glu Val Met Lys
                1605                 1610                 1615
Tyr Val Gln Lys Ile Asp Lys Glu Val Asp Lys Glu Val Ser Lys Ala
            1620                 1625                 1630
Leu Glu Ser Lys Asn Asp Val Thr Asn Val Leu Lys Gln Asn Gln Asp
        1635                 1640                 1645
Phe Phe Ser Lys Val Lys Asn Phe Val Lys Lys Tyr Lys Val Phe Ala
    1650                 1655                 1660
Ala Pro Phe Ile Ser Ala Val Ala Ala Phe Ala Ser Tyr Val Val Gly
1665                 1670                 1675                 168
Phe Phe Thr Phe Ser Leu Phe Ser Ser Cys Val Thr Ile Ala Ser Ser
                1685                 1690                 1695
Thr Tyr Leu Leu Ser Lys Val Asp Lys Thr Ile Asn Lys Asn Lys Glu
            1700                 1705                 1710
Arg Pro Phe Tyr Ser Phe Val Phe Asp Ile Phe Lys Asn Leu Lys His
        1715                 1720                 1725
Tyr Leu Gln Gln Met Lys Glu Lys Phe Ser Lys Glu Lys Asn Asn Asn
    1730                 1735                 1740
Val Ile Glu Val Thr Asn Lys Ala Glu Lys Lys Gly Asn Val Gln Val
1745                 1750                 1755                 176
Thr Asn Lys Thr Glu Lys Thr Thr Lys Val Asp Lys Asn Asn Lys Val
                1765                 1770                 1775
Pro Lys Lys Arg Arg Thr Gln Lys Ser Lys Glx
            1780                 1785

<210> 3
<211> 1712
<212> DNA
<213> T9/96 Parasite Clone

<400> 3
agtgatgaac tttttaatga attattaaat agtgtagatg ttaatggaga agtaaaagaa      60
aatattttgg aggaaagtca agttaatgac gatattttta atagtttagt aaaaagtgtt     120
caacaagaac aacaacacaa tgttgaagaa aaagttgaag aaagtgtaga agaaaatgac     180
gaagaaagtg tagaagaaaa tgtagaagaa aatgtagaag aaaatgacga cggaagtgta     240
gcctcaagtg ttgaagaaag tatagcttca agtgttgatg aaagtataga ttcaagtatt     300
gaagaaaatg tagctccaac tgttgaagaa atcgtagctc caactgttga agaaattgta     360
gctccaagtg ttgtagaaag tgtggctcca agtgttgaag aaagtgtagc tccaagtgtt     420
gaagaaagtg tagctgaaaa tgttgaagaa agtgtagctg aaaatgttga agaaatcgta     480
gctccaagtg ttgaagaaag tgtagctgaa aatgttgaag aaagtgtagc tgaaaatgtt     540
gaagaaagtg tagctgaaaa tgttgaagaa agtgtagctg aaaatgttga agaaagtgta     600
gctgaaaatg ttgaagaaat cgtagctcca actgttgaag aaagtgtagc tccaactgtt     660
gaagaaattg tagctccaac tgttgaagaa agtgtagctc caactgttga agaaattgta     720
gttccaagtg ttgaagaaag tgtagctcca agtgttgaag aaagtgtagc tgaaaatgtt     780
gaagaaagtg tagctgaaaa tgttgaagaa agtgtagctg aaaatgttga agaaagtgta     840
gctgaaaatg ttgaagaaag tgtagctgaa aatgttgaag aaatcgtagc tccaagtgtt     900
gaagaaatcg tagctccaac tgttgaagaa agtgttgctg aaaacgttgc aacaaattta     960
tcagacaatc ttttaagtaa tttattaggt ggtatcgaaa ctgaggaaat aaaggacagt    1020
atattaaatg agatagaaga agtaaaagaa aatgtagtca ccacaatact agaaaaagta    1080
gaagaaacta cagctgaaag tgtaactact tttagtaata tattagagga gatacaagaa    1140
aatactatta ctaatgatac tatagaggaa aaattagaag aactccacga aaatgtatta    1200
agtgccgctt tagaaaatac ccaaagtgaa gaggaaaaga aagaagtaat agatgtaatt    1260
gaagaagtaa aagaagaggt cgctaccact ttaatagaaa ctgtggaaca ggcagaagaa    1320
gagagcgaaa gtacaattac ggaaatattt gaaaatttag aagaaaatgc agtagaaagt    1380
aatgaaaaag ttgcagagaa tttagagaaa ttaaacgaaa ctgtatttaa tactgtatta    1440
gataaagtag aggaaacagt agaaattagc ggagaaagtt tagaaaacaa tgaaatggat    1500
aaagcatttt ttagtgaaat atttgataat gtaaaaggaa tacaagaaaa tttattaaca    1560
ggtatgtttc gaagtataga aaccagtata gtaatccaat cagaagaaaa ggttgatttg    1620
aatgaaaatg tggttagttc gattttagat aatatagaaa atatgaaaga aggtttatta    1680
aataaattag aaaatatttc aagtactgaa gg                                  1712

<210> 4
<211> 570
<212> PRT
<213> T9/96 Parasite Clone

<400> 4
Ser Asp Glu Leu Phe Asn Glu Leu Leu Asn Ser Val Asp Val Asn Gly
1               5                   10                  15
Glu Val Lys Glu Asn Ile Leu Glu Glu Ser Gln Val Asn Asp Asp Ile
            20                  25                  30
Phe Asn Ser Leu Val Lys Ser Val Gln Gln Glu Gln Gln His Asn Val
        35                  40                  45
Glu Glu Lys Val Glu Glu Ser Val Glu Glu Asn Asp Glu Glu Ser Val
    50                  55                  60
Glu Glu Asn Val Glu Glu Asn Val Glu Glu Asn Asp Asp Gly Ser Val
65                  70                  75                  80
Ala Ser Ser Val Glu Glu Ser Ile Ala Ser Ser Val Asp Glu Ser Ile
                85                  90                  95
Asp Ser Ser Ile Glu Glu Asn Val Ala Pro Thr Val Glu Glu Ile Val
            100                 105                 110
Ala Pro Thr Val Glu Glu Ile Val Ala Pro Ser Val Val Glu Ser Val
        115                 120                 125
Ala Pro Ser Val Glu Glu Ser Val Ala Pro Ser Val Glu Glu Ser Val
    130                 135                 140
Ala Glu Asn Val Glu Glu Ser Val Ala Glu Asn Val Glu Glu Ile Val
145                 150                 155                 160
Ala Pro Ser Val Glu Glu Ser Val Ala Glu Asn Val Glu Glu Ser Val
                165                 170                 175
Ala Glu Asn Val Glu Glu Ser Val Ala Glu Asn Val Glu Glu Ser Val
            180                 185                 190
Ala Glu Asn Val Glu Glu Ser Val Ala Glu Asn Val Glu Glu Ile Val
        195                 200                 205
Ala Pro Thr Val Glu Glu Ser Val Ala Pro Thr Val Glu Glu Ile Val
    210                 215                 220
Ala Pro Thr Val Glu Glu Ser Val Ala Pro Thr Val Glu Glu Ile Val
225                 230                 235                 240
Val Pro Ser Val Glu Glu Ser Val Ala Pro Ser Val Glu Glu Ser Val
                245                 250                 255
Ala Glu Asn Val Glu Glu Ser Val Ala Glu Asn Val Glu Glu Ser Val
            260                 265                 270
Ala Glu Asn Val Glu Glu Ser Val Ala Glu Asn Val Glu Glu Ser Val
        275                 280                 285
Ala Glu Asn Val Glu Glu Ile Val Ala Pro Ser Val Glu Glu Ile Val
    290                 295                 300
Ala Pro Thr Val Glu Glu Ser Val Ala Glu Asn Val Ala Thr Asn Leu
305                 310                 315                 320
Ser Asp Asn Leu Leu Ser Asn Leu Leu Gly Gly Ile Glu Thr Glu Glu
                325                 330                 335
Ile Lys Asp Ser Ile Leu Asn Glu Ile Glu Glu Val Lys Glu Asn Val
            340                 345                 350
Val Thr Thr Ile Leu Glu Lys Val Glu Glu Thr Thr Ala Glu Ser Val
        355                 360                 365
Thr Thr Phe Ser Asn Ile Leu Glu Glu Ile Gln Glu Asn Thr Ile Thr
    370                 375                 380
Asn Asp Thr Ile Glu Glu Lys Leu Glu Glu Leu His Glu Asn Val Leu
385                 390                 395                 400
Ser Ala Ala Leu Glu Asn Thr Gln Ser Glu Glu Glu Lys Lys Glu Val
                405                 410                 415
Ile Asp Val Ile Glu Glu Val Lys Glu Glu Val Ala Thr Thr Leu Ile
            420                 425                 430
Glu Thr Val Glu Gln Ala Glu Glu Glu Ser Glu Ser Thr Ile Thr Glu
        435                 440                 445
Ile Phe Glu Asn Leu Glu Glu Asn Ala Val Glu Ser Asn Glu Lys Val
    450                 455                 460

Ala Glu Asn Leu Glu Lys Leu Asn Glu Thr Val Phe Asn Thr Val Leu
465                 470                 475                 480
Asp Lys Val Glu Glu Thr Val Glu Ile Ser Gly Glu Ser Leu Glu Asn
                485                 490                 495
Asn Glu Met Asp Lys Ala Phe Phe Ser Glu Ile Phe Asp Asn Val Lys
            500                 505                 510
Gly Ile Gln Glu Asn Leu Leu Thr Gly Met Phe Arg Ser Ile Glu Thr
        515                 520                 525
Ser Ile Val Ile Gln Ser Glu Glu Lys Val Asp Leu Asn Glu Asn Val
    530                 535                 540
Val Ser Ser Ile Leu Asp Asn Ile Glu Asn Met Lys Glu Gly Leu Leu
545                 550                 555                 560
Asn Lys Leu Glu Asn Ile Ser Ser Thr Glu
                565                 570

<210> 5
<211> 20
<212> PRT
<213> Artificial Sequence

<220>
<223> Synthetic peptide

<400> 5
Leu Leu Ser Asn Ile Glu Glu Pro Lys Glu Asn Ile Ile Asp Asn Leu
1               5                   10                  15
Leu Asn Asn Ile
            20

<210> 6
<211> 25
<212> PRT
<213> Artificial Sequence

<220>
<223> synthetic peptide

<400> 6
Asp Glu Leu Phe Asn Glu Leu Leu Asn Ser Val Asp Val Asn Gly Glu
1               5                   10                  15
Val Lys Glu Asn Ile Leu Glu Glu Ser
            20                  25

<210> 7
<211> 26
<212> PRT
<213> Artificial Sequence

<220>
<223> synthetic peptide

<400> 7
Leu Glu Glu Ser Gln Val Asn Asp Asp Ile Phe Asn Ser Leu Val Lys
1               5                   10                  15
Ser Val Gln Gln Glu Gln Gln His Asn Val
            20                  25

<210> 8
<211> 28
<212> PRT
<213> Artificial Sequence

<220>
<223> synthetic peptide

<400> 8
Val Glu Ser Val Ala Pro Ser Val Glu Glu Ser Val Ala Pro Ser Val
1               5                   10                  15
Glu Glu Ser Val Ala Glu Asn Val Glu Glu Ser Val
            20                  25